# EUROPEAN PATENT APPLICATION

(11) **EP 2 735 273 A1**
(43) Date of publication of application: **28.05.2014**
(21) Application number: 12815219.6
(22) Date of filing: 04.04.2012
(51) Int. Cl.: A61B 10/02

(54) **INSTRUMENT FOR COLLECTING BODY TISSUE AND METHOD FOR COLLECTING BODY TISSUE USING SAME**

(30) Priority: 21.07.2011 JP 2011159660; 08.09.2011 JP 2011195823
(71) Applicant: Panasonic Healthcare Co., Ltd., Ehime 791-0395 (JP)
(72) Inventor: FUKUHARA, Takaomi, Osaka 540-6207 (JP); NANJO, Toshifumi, Osaka 540-6207 (JP); KITAWAKI, Fumihisa, Osaka 540-6207 (JP)
(74) Representative: TBK
(86) International application number: PCT/JP2012/002365
(87) International publication number: WO 2013/011607

(57) **Abstract**

Provided is a biological tissue collecting tool capable of reducing a variation in the amount of collected biological tissue at the time of collecting the biological tissue from a biological aperture. A biological tissue collecting tool 10 according to the present invention includes: an exterior body 3 having first space formed therein and having a first collection opening 2 formed in a wall thereof, the exterior body 3 having such a shape as to be insertable in a biological aperture; a collecting portion 15 disposed in the first space such that the collecting portion 15 is movable in the first space, the collecting portion 15 being configured to collect a part of biological tissue that protrudes into the first space through the first collection opening 2; and an operating portion 7 connected to the collecting portion 15, the operating portion 7 allowing the collecting portion 15 to be operated from outside of the exterior body 3.

## Description

### Technical Field

The present invention relates to a biological tissue collecting tool and a biological tissue collection method using the same.

### Background Art

Cotton swabs are widely used as a tool for collecting biological tissue from a biological aperture. For example, a cotton swab is used to collect a mucous membrane from the inside of a nostril, and then the collected mucous membrane is used to perform a simple flu test with a flue test kit. Such a simple test merely requires collection of a part of biological tissue, and whether the amount of collected biological tissue is large or small does not particularly matter.

Meanwhile, there are test methods where biological tissue is to be collected in a substantially fixed amount, and the collected biological tissue is used to determine a health state, for example. In a case where such a test method is used, collection of biological tissue in the aforementioned manner using a cotton swab is unfavorable since the amount of biological tissue to be collected by the cotton swab varies.

In view of the above, it has been proposed to mark a cotton swab with a scale, or to form a cotton swab such that the cotton swab can be cut into a predetermined length (see Patent Literature 1, for example).

### Citation List

### Patent Literature

PTL 1: Japanese Laid-Open Patent Application Publication No. 2001-289844

### Summary of Invention

### Technical Problem

The above-described conventional art has a problem in that the accuracy of a determination made in a test cannot be improved.

To be specific, it was expected that, with the use of a cotton swab marked with a scale or a cotton swab formed such that the cotton swab can be cut into a predetermined length, the amount of collected biological tissue should be substantially fixed each time the tissue collection was performed. However, when a cotton swab is used to collect biological tissue, the biological tissue is collected by utilizing its adhesion to the cotton swab, and it is difficult to keep the tissue adhesion amount to a fixed amount each time the tissue collection is performed. As a result, even if the aforementioned types of cotton swabs are used, the amount of collected biological tissue significantly varies each time the tissue collection is performed. For this reason, conventionally, the accuracy of a determination made in a test cannot be improved.

In view of the above, an object of the present invention is to reduce a variation in the amount of collected biological tissue, thereby improving the accuracy of a determination made in a test.

### Solution to Problem

In order to achieve the above object, the present invention relates to a biological tissue collecting tool including: an exterior body having first space formed therein and having a first collection opening formed in a wall thereof, the exterior body having such a shape as to be insertable in a biological aperture; a collecting portion disposed in the first space such that the collecting portion is movable in the first space, the collecting portion being configured to collect a part of biological tissue that protrudes into the first space through the first collection opening; and an operating portion connected to the collecting portion, the operating portion allowing the collecting portion to be operated from outside of the exterior body.

### Advantageous Effects of Invention

According to the present invention, a substantially fixed amount of biological tissue can be collected each time biological tissue is collected from a biological aperture, and therefore, a variation in the amount of collected biological tissue can be reduced. This consequently makes it possible to improve the accuracy of a determination made in a test.

### Brief Description of Drawings

[Fig. 1] Fig. 1 is a conceptual diagram showing a biological tissue collection method using a biological tissue collecting tool 10 according to Embodiment 1.
[Fig. 2] Fig. 2 is a perspective view showing the biological tissue collecting tool 10 according to Embodiment 1.
[Fig. 3A] Fig. 3A is a perspective view showing an exterior body of Fig. 2.
[Fig. 3B] Fig. 3B is a perspective view showing an interior body of Fig. 2.
[Fig. 3C] Fig. 3C is a perspective view showing an operated body of Fig. 2.
[Fig. 4] Fig. 4 is a perspective view showing a biological tissue collecting tool 20 according to Embodiment 2.
[Fig. 5A] Fig. 5A is a perspective view showing an operated body of Fig. 4.
[Fig. 5B] Fig. 5B is a perspective view showing an interior body of Fig. 4.
[Fig. 5C] Fig. 5C is a perspective view showing an exterior body of Fig. 4.
[Fig. 6] Fig. 6 is a perspective view showing an operated body 4 of the biological tissue collecting tool 20 according to Embodiment 2.
[Fig. 7] Fig. 7 is a cross-sectional view showing the vicinity of a first collection opening 2 when the biological tissue collecting tool 20 according to Embodiment 2 is inserted in a nostril.
[Fig. 8] Fig. 8 is a cross-sectional view showing the vicinity of the first collection opening 2 when the biological tissue collecting tool 20 according to Embodiment 2 is inserted in the nostril.
[Fig. 9] Fig. 9 is a cross-sectional view showing the vicinity of the first collection opening 2 when the biological tissue collecting tool 20 according to Embodiment 2 is inserted in the nostril.
[Fig. 10] Fig. 10 is a cross-sectional view showing the vicinity of the first collection opening 2 when the biological tissue collecting tool 20 according to Embodiment 2 is inserted in the nostril.
[Fig. 11] Fig. 11 is a cross-sectional view showing the vicinity of the first collection opening 2 when the biological tissue collecting tool 20 according to Embodiment 2 is inserted in the nostril.
[Fig. 12A] Fig. 12A is a perspective view showing a biological tissue collecting tool 30 according to Embodiment 3 in an opened state.
[Fig. 12B] Fig. 12B is a perspective view showing the biological tissue collecting tool 30 according to Embodiment 3 in a closed state.
[Fig. 13A] Fig. 13A is a front view of a first collection opening 22 of the biological tissue collecting tool 30 according to Embodiment 3 in an opened state.
[Fig. 13B] Fig. 13B is a front view of the first collection opening 22 of the biological tissue collecting tool 30 according to Embodiment 3 in a closed state.
[Fig. 14A] Fig. 14A is a cross-sectional view of the biological tissue collecting tool 30 according to Embodiment 3 in an opened state, the cross-sectional view being perpendicular to an operating stick 26 and including the first collection opening 22.
[Fig. 14B] Fig. 14B is a cross-sectional view of the biological tissue collecting tool 30 according to Embodiment 3 in a closed state, the cross-sectional view being perpendicular to the operating stick 26 and including the first collection opening 22.
[Fig. 15A] Fig. 15A is a cross-sectional view of the biological tissue collecting tool 30 according to Embodiment 3 in an opened state, the cross-sectional view including the operating stick 26 and the first collection opening 22.
[Fig. 15B] Fig. 15B is a cross-sectional view of the biological tissue collecting tool 30 according to Embodiment 3 in a closed state, the cross-sectional view including the operating stick 26 and the first collection opening 22.
[Fig. 16] Fig. 16 is a cross-sectional view of the biological tissue collecting tool 30 according to Embodiment 3 in a closed state, which is inserted in a nostril, the cross-sectional view being perpendicular to the operating stick 26 and including the first collection opening 22.
[Fig. 17A] Fig. 17A shows the biological tissue collecting tool 30 according to Embodiment 3 whose state has transitioned from the state shown in Fig. 16 to an opened state.
[Fig. 17B] Fig. 17B is an enlarged view showing a relationship between a mucous membrane and the edge of a third collection opening 24.
[Fig. 17C] Fig. 17C is an enlarged view showing a situation when the mucous membrane is cut off by the third collection opening 24.
[Fig. 18] Fig. 18 illustrates an example of a method of subjecting the mucous membrane collected by the biological tissue collecting tool 30 according to Embodiment 3 to a test.
[Fig. 19A] Fig. 19A shows a liquid being flowed into the biological tissue collecting tool 30 according to Embodiment 3 in another example of a method of subjecting the mucous membrane collected by the biological tissue collecting tool 30 according to Embodiment 3 to a test.
[Fig. 19B] Fig. 19B shows the biological tissue collecting tool 30 according to Embodiment 3 being shaken in a closed state in yet another example of a method of subjecting the mucous membrane collected by the biological tissue collecting tool 30 according to Embodiment 3 to a test.
[Fig. 20] Fig. 20 is a cross-sectional view of a biological tissue collecting tool 40 according to Embodiment 4 in an opened state, the cross-sectional view being perpendicular to the operating stick 26 and including the first collection opening 22.
[Fig. 21] Fig. 21 is a perspective view showing a biological tissue collecting tool 50 according to Embodiment 5.
[Fig. 22] Fig. 22 is a perspective view showing a state where a plurality of biological tissue collecting tools 30 according to Embodiment 3 are arranged for storage.
[Fig. 23A] Fig. 23A is a perspective view showing the biological tissue collecting tool 30 according to Embodiment 3 in combination with an operating tool.
[Fig. 23B] Fig. 23B is a transparent perspective view showing the biological tissue collecting tool 30 according to Embodiment 3 in combination with the operating tool.
[Fig. 24] Fig. 24 is a perspective view showing a biological tissue collecting tool 60 according to Embodiment 6 together with a hand.
[Fig. 25] Fig. 25 shows a state where the biological tissue collecting tool 60 according to Embodiment 6 is inserted in a nostril.
[Fig. 26A] Fig. 26A is a cross-sectional view of the biological tissue collecting tool 60 according to Embodiment 6 in an opened state, the cross-sectional view including the operating stick 26 and the first collection opening 22.
[Fig. 26B] Fig. 26B is a cross-sectional view of the biological tissue collecting tool 60 according to Embodiment 6 in a closed state, the cross-sectional view including the operating stick 26 and the first collection opening 22.
[Fig. 27] Fig. 27 is a perspective view showing the biological tissue collecting tool 60 according to Embodiment 6 in another mode.
[Fig. 28] Fig. 28 is a perspective view showing the biological tissue collecting tool 60 according to Embodiment 6 in yet another mode.
[Fig. 29] Fig. 29 is a perspective view showing a biological tissue collecting tool 70 according to Embodiment 7 together with a hand.
[Fig. 30] Fig. 30 shows a state where the biological tissue collecting tool 70 according to Embodiment 7 is inserted in a nostril.
[Fig. 31] Fig. 31 shows a state where a wipe cover 61 is pulled backward while the biological tissue collecting tool 70 according to Embodiment 7 is kept inserted in the nostril.
[Fig. 32] Fig. 32 shows a state where the wipe cover 61 of the biological tissue collecting tool 70 according to Embodiment 7 is further pulled backward.
[Fig. 33] Fig. 33 shows a state where the wipe cover 61 is removed from the biological tissue collecting tool 70 according to Embodiment 7.
[Fig. 34A] Fig. 34A is a cross-sectional view of a biological tissue collecting tool 80 according to Embodiment 8 in a closed state prior to tissue collection, the cross-sectional view being perpendicular to the operating stick 26 and including the first collection opening 22.
[Fig. 34B] Fig. 34B is a cross-sectional view of the biological tissue collecting tool 80 according to Embodiment 8 in an opened state, the cross-sectional view being perpendicular to the operating stick 26 and including the first collection opening 22.
[Fig. 34C] Fig. 34C is a cross-sectional view of the biological tissue collecting tool 80 according to Embodiment 8 being in a transition from an opened state to a closed state, the cross-sectional view being perpendicular to the operating stick 26 and including the first collection opening 22.
[Fig. 34D] Fig. 34D is a cross-sectional view of the biological tissue collecting tool 80 according to Embodiment 8 in a closed state after tissue collection, the cross-sectional view being perpendicular to the operating stick 26 and including the first collection opening 22.
[Fig. 35A] Fig. 35A is a cross-sectional view of a biological tissue collecting tool 90 according to Embodiment 9 in a closed state prior to tissue collection, the cross-sectional view being perpendicular to the operating stick 26 and including the first collection opening 22.
[Fig. 35B] Fig. 35B is a cross-sectional view of the biological tissue collecting tool 90 according to Embodiment 9 in an opened state, the cross-sectional view being perpendicular to the operating stick 26 and including the first collection opening 22.
[Fig. 35C] Fig. 35C is a cross-sectional view of the biological tissue collecting tool 90 according to Embodiment 9 being in a transition from an opened state to a closed state, the cross-sectional view being perpendicular to the operating stick 26 and including the first collection opening 22.
[Fig. 35D] Fig. 35D is a cross-sectional view of the biological tissue collecting tool 90 according to Embodiment 9 in a closed state after tissue collection, the cross-sectional view being perpendicular to the operating stick 26 and including the first collection opening 22.
[Fig. 36] Fig. 36 is a perspective view showing a biological tissue collecting tool 100 according to Embodiment 10.
[Fig. 37A] Fig. 37A shows the biological tissue collecting tool 100 according to Embodiment 10 in which a reagent body 81 has not been moved yet.
[Fig. 37B] Fig. 37B shows the biological tissue collecting tool 100 according to Embodiment 10 in which the reagent body 81 has been moved.
[Fig. 38] Fig. 38 illustrates a reaction confirmation window 85 of the biological tissue collecting tool 100 according to Embodiment 10, including: (a) an overall view; (b) an enlarged view of the vicinity of an exterior case 21; and (c) an enlarged view of the vicinity of the reaction confirmation window 85.
[Fig. 39] Fig. 39 illustrates an example of a method of subjecting a mucous membrane collected by a biological tissue collecting tool 110 according to Embodiment 11 to a test.

### Description of Embodiments

Hereinafter, embodiments of the present invention are described in detail with reference to the drawings.

### (Embodiment 1)

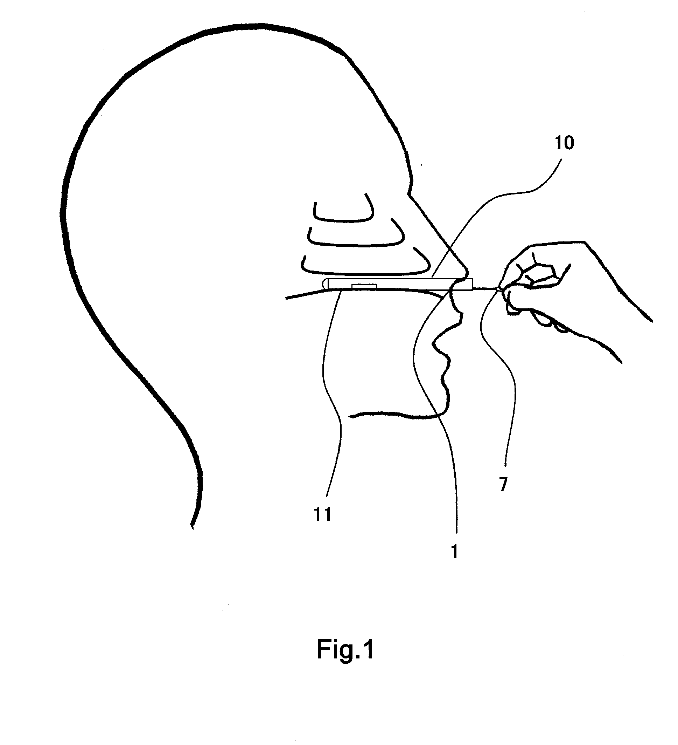

Fig. 1 shows a biological tissue collection method using a biological tissue collecting tool 10 according to Embodiment 1.

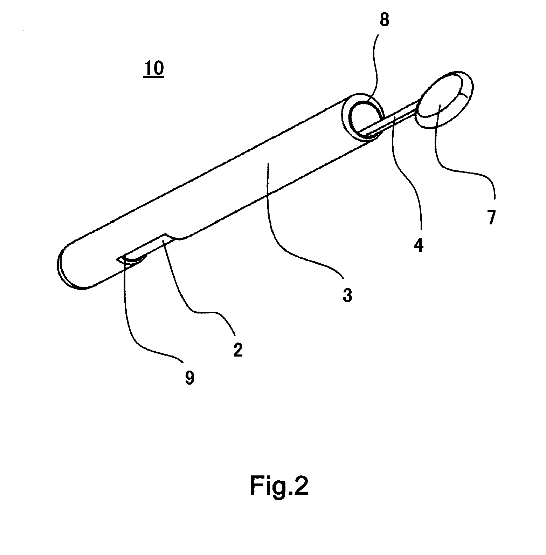

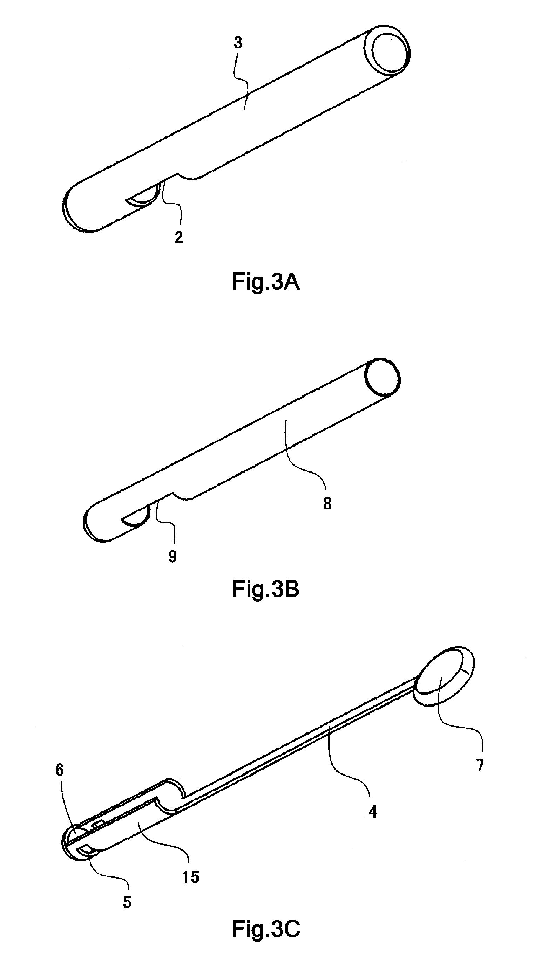

As shown in Fig. 2 and Figs. 3A to 3C, the biological tissue collecting tool 10 according to the present embodiment includes: a cylindrical exterior body 3 having such a shape as to be insertable in a biological aperture; a cylindrical interior body 8 disposed within the exterior body so as to be in close contact with the exterior body; and an operated body 4 movably disposed within the exterior and interior bodies.

The exterior body 3 has a first collection opening 2 in its side wall, and has first space formed therein. The overall shape of the exterior body 3 is such a cylindrical shape as to be insertable into a biological aperture, and the exterior body 3 has a closed end and an open end. The exterior body 3 is formed of a soft material such as a silicone, and the hardness of the soft material is less than or equal to 30°, and preferably in a range from 5° to 10°. That is, the exterior body 3 is formed of a very soft material. The hardness is measured by a former JIS K 6301 spring-type hardness test.

The interior body 8 has such a cylindrical shape as to be fitted in the first space of the exterior body 3, and has a closed end and an open end. The interior body 8 is inserted in the exterior body, such that the closed end of the exterior body 3 and the closed end of the interior body 8 face in the same direction. In a state where the interior body 8 is inserted in the exterior body 3, the inner wall of the exterior body 3 and the outer wall of the interior body 8 are in close contact with each other. The interior body 8 has a second collection opening 9 in its side wall, and has second space formed therein. The second space has such a shape as to allow a collecting portion 15 to be movably inserted therein. The second collection opening 9 has substantially the same shape as that of the first collection opening 2, and is provided such that the position of the second collection opening 9 overlaps the position of the first collection opening 2.

The interior body 8 is formed of a material (e.g., a synthetic resin) harder than the material of the exterior body 3. Since the exterior body 3 is formed of a soft material as described above, the interior body 8 formed of a hard material is disposed within the exterior body 3 for the purpose of maintaining the shape of the exterior body 3 and making it possible to readily operate the operated body 4 within the exterior body 3 in the axial direction of the cylindrical exterior body 3.

The operated body 4 has the collecting portion 15 at its one end and an operating portion 7 at its other end, the other end being positioned at the opposite side to the one end provided with the collecting portion 15. The operating portion 7 allows the collecting portion 15 to be operated from the outside of the exterior body 3. The operating portion 7 has a shape suitable to be held by finger tips or by a hand. As shown in Fig. 2, the operating portion 7 is exposed at the outside of the exterior body 3 and the interior body 8. Specifically, the operating portion 7 protrudes to the outside of the exterior body 3 and the interior body 8 through the open end of the exterior body 3 and the open end of the interior body 8.

The collecting portion 15 is configured to collect a part of biological tissue by being operated by means of the operating portion from the outside of the exterior body 3 and the interior body 8. The biological tissue to be collected is in a state of protruding into the first space of the exterior body 3 through the first collection opening 2 of the exterior body 3 and the second collection opening 9 of the interior body 8. The collecting portion 15 has a side wall being in close contact with the inner wall of the interior body 8, and a third collection opening 5 is formed in the side wall. Further, the collecting portion 15 has a storage portion 6 at its one end. The storage portion 6 temporarily stores the collected biological tissue. The storage portion 6 is formed so as to be in communication with the third collection opening 5. The side wall of the collecting portion 15 and the operating portion 7 are connected by a rod-like member. The third collection opening 5 is provided so as to be positioned closer to the closed ends of the exterior body 3 and the interior body 8 than the first collection opening 2 and the second collection opening 9.

The operated body 4 is movably inserted in the first space of the exterior body 3 or the second space of the interior body 8. Specifically, the operated body 4 can slidingly move within the second space in the interior body 8 in the axial direction of the cylindrical exterior body 3 and the cylindrical interior body 8.

Hereinafter, with reference to Fig. 1, a description is given of an example in which the biological tissue collecting tool 10 having the above-described configuration is used to collect a part of tissue in a common nasal meatus at the back of an external nostril.

First, the biological tissue collecting tool 10 is in a state where the operated body 4 is pushed in the interior body 8 such that the operated body 4 is in contact with the inner wall of the interior body 8 at the closed end. Then, the biological tissue collecting tool 10 in such a state is inserted from an external nostril 1 into a common nasal meatus 11 as shown in Fig. 1. Next, the operating portion 7 is operated, such that the operated body 4 is pulled and moved in the axial direction of the cylindrical exterior body 3 toward the outside of the external nostril 1.

During such an operation, when the third collection opening 5 of the operated body 4 is in a state of overlapping the first collection opening 2 of the exterior body 3 and the second collection opening 9 of the interior body 8, a part of the tissue of the common nasal meatus 11 is in a state of protruding into the biological tissue collecting tool 10 through the first collection opening 2 of the exterior body 3, the second collection opening 9 of the interior body 8, and the third collection opening 5 of the operated body 4.

When the operated body 4 in the above state is further moved toward the outside of the external nostril 1, the rear edge (i.e., an edge at the closed end side) of the third collection opening 5 of the operated body 4 passes through the first collection opening 2 of the exterior body 3 and the second collection opening 9 of the interior body 8. Accordingly, the rear edge of the third collection opening 5 serves as a cutting portion that scratches away the tissue of the common nasal meatus 11, the tissue protruding into the biological tissue collecting tool 10, and then the tissue obtained by the scratching is stored in the storage portion 6.

In this state, the biological tissue collecting tool 10 is entirely pulled out of the external nostril 1, and thus the biological tissue collection is completed. The biological tissue collected by the biological tissue collecting tool 10 can be directly used in a predetermined test. Alternatively, the operated body 4 may be removed from the biological tissue collecting tool 10; then the collecting portion 15 may be immersed in a liquid and shaken such that the collected biological tissue is dissolved into the liquid; and thereafter a resultant solution may be used in a predetermined test.

The exterior body 3 of the biological tissue collecting tool 10 is formed of a soft material, and therefore, even when the exterior body 3 is in close contact with the surface of the wall of the common nasal meatus 11, the exterior body 3 is less likely to irritate the common nasal meatus 11, i.e., less likely to cause sneezing or the like. Therefore, the above-described tissue collection can be performed very easily.

Although the exterior body 3 is cylindrical, the exterior body 3 is formed such that, as shown in Fig. 2 and Figs. 3A to 3C, the wall of the exterior body at the opposite side to the first collection opening 2 is thicker than the wall of the exterior body that is positioned above and below the first collection opening 2 with respect to the axial direction of the exterior body. Such a structure allows, when the biological tissue collecting tool 10 is inserted into the common nasal meatus 11, the thicker wall to be pushed by the inner surface of the common nasal meatus 11, and as a result, the first collection opening 2 is pushed against the inner surface of the common nasal meatus 11. This facilitates the protrusion of a part of the tissue of the common nasal meatus 11 into the biological tissue collecting tool 10 through the first collection opening 2 and the second collection opening 9.

The exterior body 3 of the biological tissue collecting tool 10 is formed of a soft silicone, and the operated body 4 and the interior body 8 of the biological tissue collecting tool 10 are formed of a hard resin. Therefore, even if the biological tissue collecting tool 10, after having collected the biological tissue, is directly immersed in a liquid in a test tube and shaken such that the collected biological tissue is dissolved into the liquid, no alteration of the collected biological tissue occurs.

According to the present embodiment, the tissue of the common nasal meatus 11 that protrudes into the biological tissue collecting tool 10 through the first collection opening 2 of the exterior body 3 and the second collection opening 9 of the interior body 8 is collected. Therefore, a substantially fixed amount of tissue can be collected each time the tissue collection is performed. That is, with the use of the biological tissue collecting tool 10, a test using biological tissue collected in a fixed amount can be performed as necessary, and the accuracy of a determination made in the test can be improved.

### (Embodiment 2)

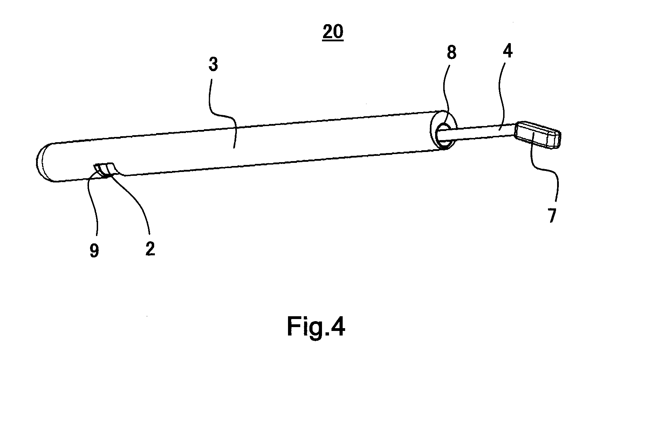

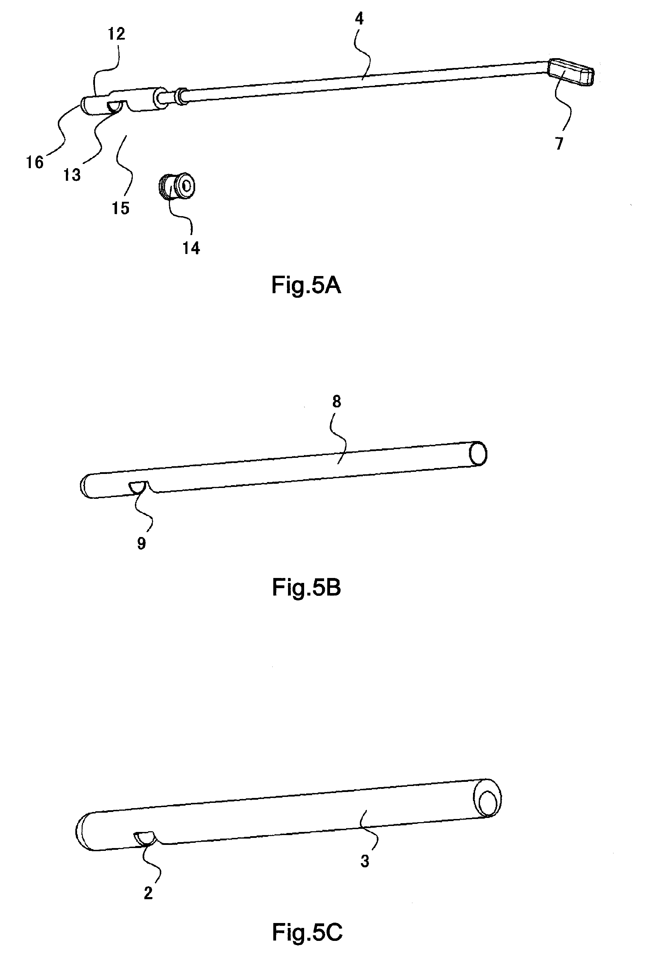

Fig. 4, Figs. 5A to 5C, and Fig. 6 show a biological tissue collecting tool 20 according to Embodiment 2. The biological tissue collecting tool according to the present embodiment includes: the cylindrical exterior body 3 having such a shape as to be insertable in a biological aperture; the cylindrical interior body 8 disposed within the exterior body so as to be in close contact with the exterior body; and an operated body 4 movably disposed within the exterior and interior bodies. Since the exterior body 3 and the interior body 8 are the same as those described in Embodiment 1, a description thereof is omitted.

The operated body 4 has a collecting portion 15 at its one end. The collecting portion 15 is for use in collecting a part of biological tissue that protrudes into the first space of the exterior body 3 through the first collection opening 2 of the exterior body 3 and the second collection opening 9 of the interior body 8. Further, the operated body 4 has an operating portion 7 at its other end, the other end being positioned at the opposite side to the one end provided with the collecting portion 15. The operating portion 7 allows the collecting portion 15 to be operated from the outside of the exterior body 3. The operating portion 7 has a shape suitable to be held by finger tips or by a hand. As shown in Fig. 4, the operating portion 7 protrudes to the outside of the exterior body 3 and the interior body 8 through the open end of the exterior body 3 and the open end of the interior body 8.

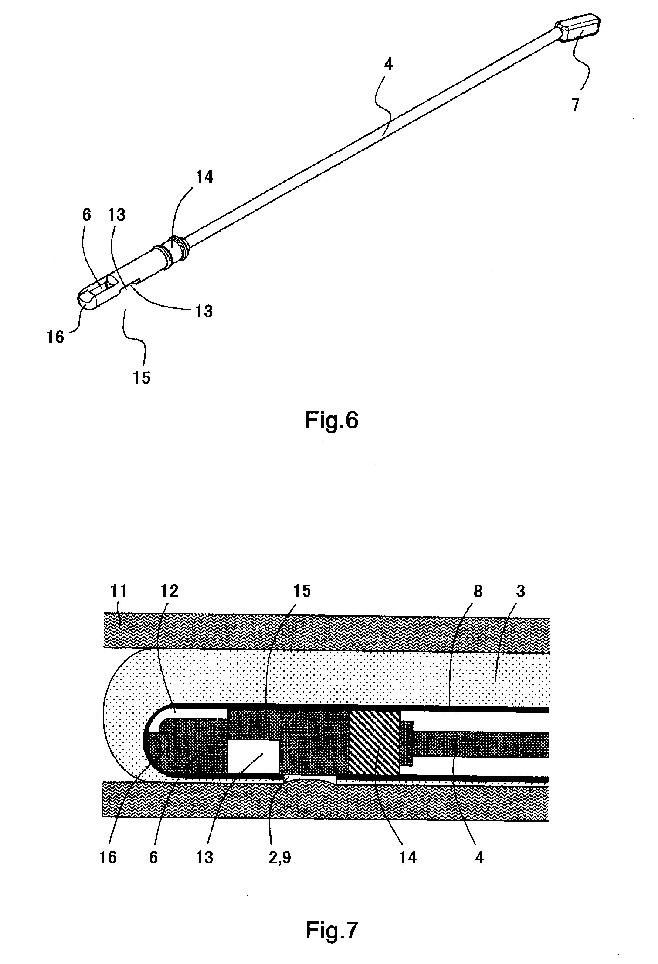

The collecting portion 15 is substantially columnar and has a side wall whose shape is fitted to the shape of the inner wall of the interior body 8, and a third collection opening 13 is formed in the side wall. Further, the collecting portion 15 has, at its one end, a contacting portion 16 configured to come into contact with the inner wall of the interior body 8 at the closed end. The third collection opening 13 is in communication with the interior of the contacting portion 16, thus forming space. The space serves as a storage portion 6 for temporarily storing collected biological tissue. Further, the storage portion 6 is in communication with upper space 12 above the contacting portion 16. The upper space 12 is space that is formed by cutting off a part of an end portion of the collecting portion 15. The collecting portion 15 and the operating portion 7 are connected by a rod-like member. As shown in Fig. 7, the third collection opening 13 is provided so as to be positioned closer to the closed ends of the exterior body 3 and the interior body 8 than the first collection opening 2 and the second collection opening 9.

Further, packing 14 is disposed at a boundary between the collecting portion 15 and the rod-like member so as to be in contact with the inner wall surface of the interior body. By disposing the packing 14 between the collecting portion 15 and the operating portion 7 in this manner, space between the packing and the closed end of the interior body can be sealed up.

The operated body 4 is movably inserted in the first space of the exterior body 3 or the second space of the interior body 8. Specifically, the operated body 4 can slidingly move in the axial direction of the cylindrical exterior body 3 and the cylindrical interior body 8.

The biological tissue collecting tool 20 according to the present embodiment may be used in the same manner as the biological tissue collecting tool 10 according to Embodiment 1, and thereby a part of tissue in the common nasal meatus at the back of an external nostril can be collected. Hereinafter, differences from a case where the biological tissue collecting tool 10 is used are described.

When the biological tissue collecting tool 20 is in a state where the operated body 4 is pushed in the interior body 8 such that the operated body 4 is in contact with the inner wall of the interior body 8 at the closed end, the contacting portion 16 of the operated body 4 is, as shown in Fig. 7, in contact with the inner wall of the interior body 8 at the closed end.

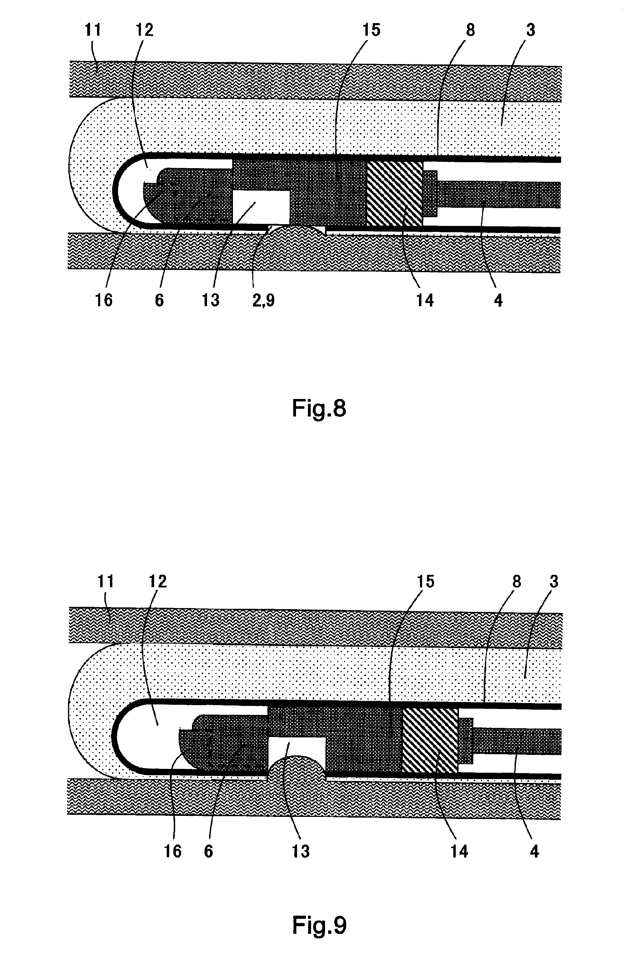

In this state, the operating portion 7 is operated such that the operated body 4 is pulled and moved in the axial direction of the cylindrical exterior body 3 toward the outside of the external nostril 1. As a result, the upper space 12 between the contacting portion 16 and the interior body gradually expands as shown in Fig. 8 to Fig. 11. At the time, the upper space 12 is caused to be under negative pressure since the packing 14 is disposed outside the collecting portion 15. Accordingly, as shown in Fig. 8, a part of the tissue of the common nasal meatus 11 passes through the first collection opening 2 of the exterior body 3 and the second collection opening 9 of the interior body 8, and protrudes into the second space of the interior body 8. Further, as shown in Fig. 9, when the third collection opening 13 of the operated body 4 is in a state of overlapping the first collection opening 2 of the exterior body 3 and the second collection opening 9 of the interior body 8, a part of the tissue of the common nasal meatus 11 is in a state of protruding to the inside through the third collection opening 13 of the collecting portion 15. At the time, the protrusion of the tissue is facilitated due to the upper space 12 being under negative pressure.

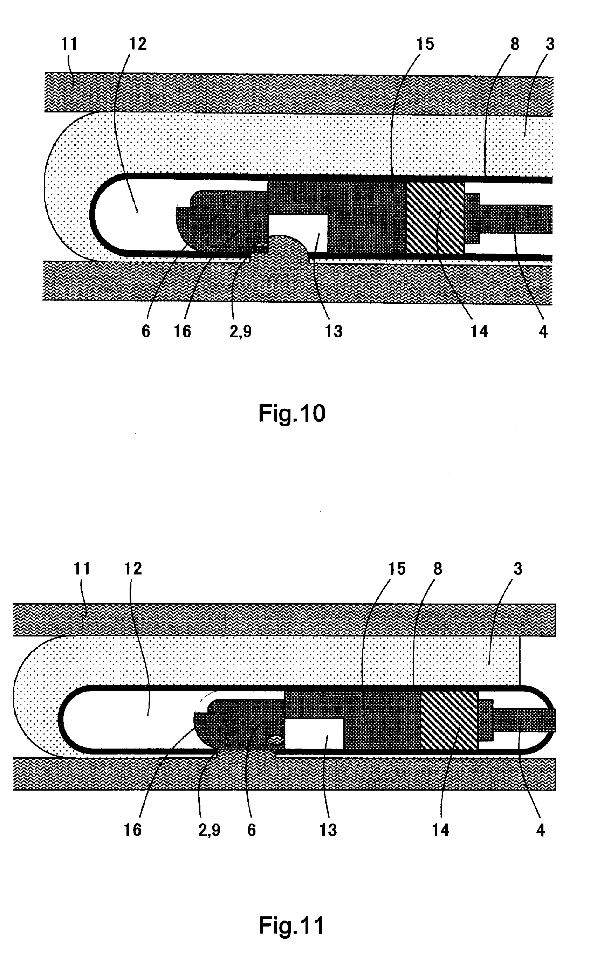

When the operated body 4 in the above state is further pulled and moved toward the outside of the external nostril 1 as shown in Fig. 10 and Fig. 11, the rear edge of the third collection opening 13 of the operated body 4 scratches away the protruding tissue of the common nasal meatus, and then the tissue is stored in the storage portion 6. When the biological tissue collected in this manner is in a state of being stored in the storage portion 6, there is not a risk that the biological tissue inadvertently flows out of the exterior body 3 since the first collection opening 2 and the second collection opening 9 are covered by the wall of the collecting portion 15. Moreover, the contacting portion is positioned below the third collection opening 13 at one end of the storage portion 6, and thereby a so-called pocket is formed, which assuredly prevents the inadvertent outflow of the biological tissue from the exterior body 3.

### (Embodiment 3)

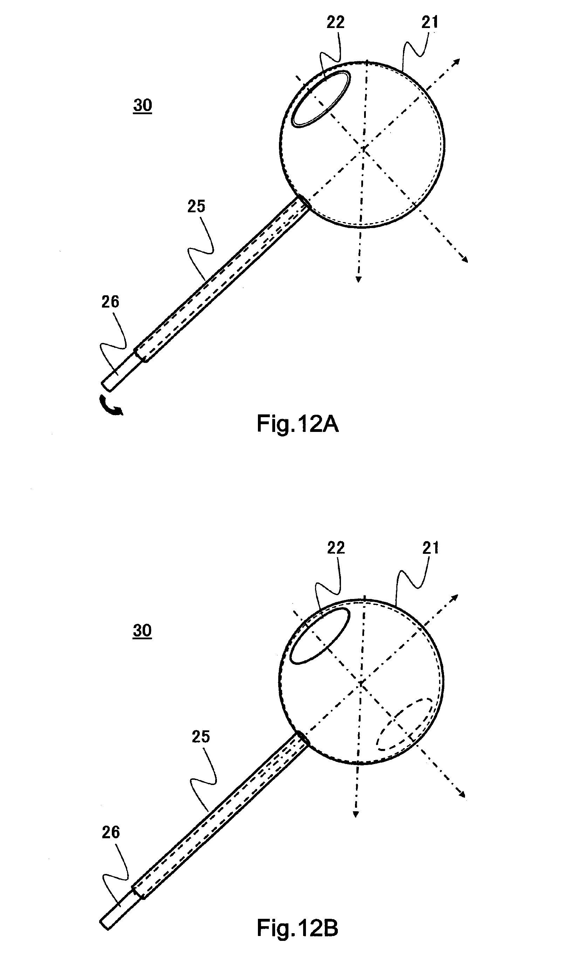

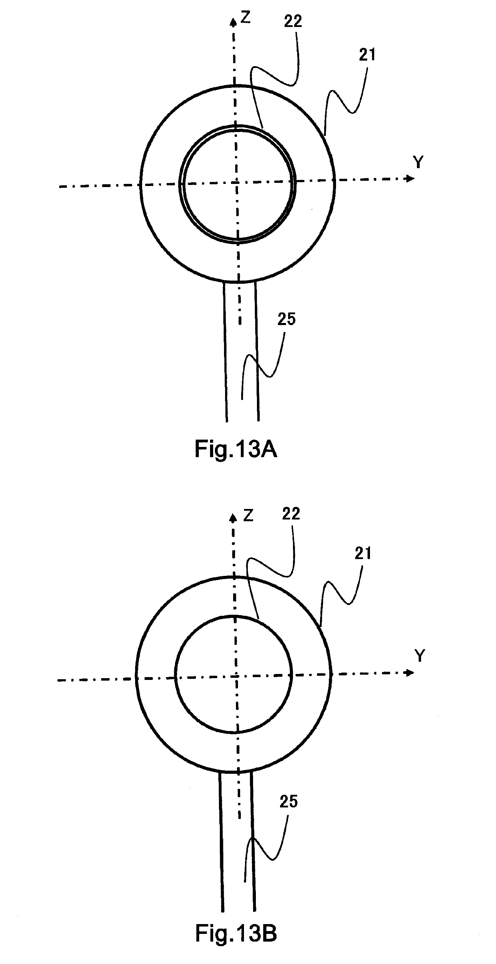

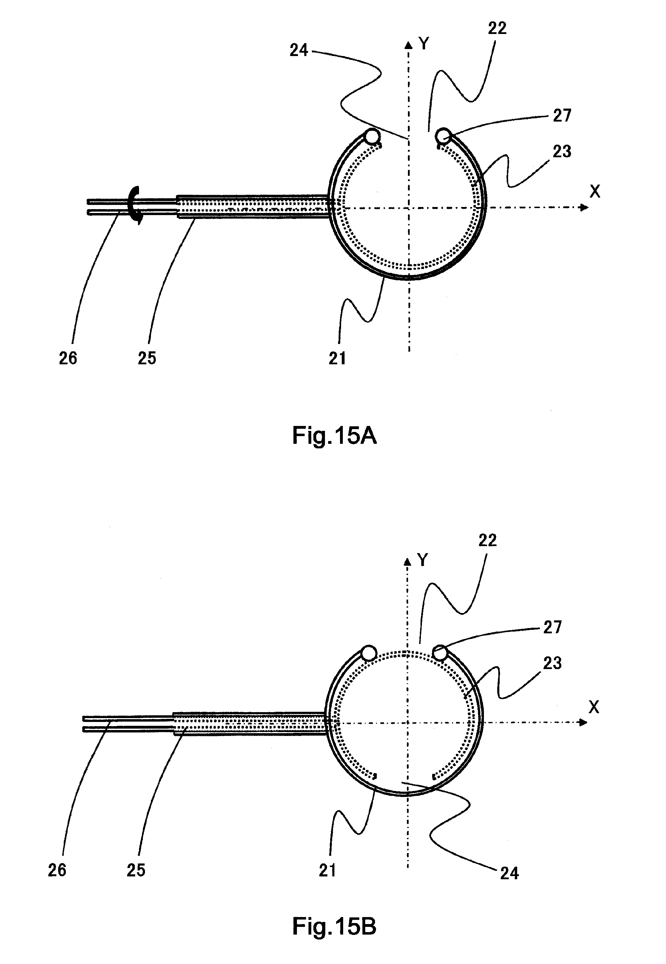

Fig. 12A to Fig. 15B show a biological tissue collecting tool 30 according to Embodiment 3. The biological tissue collecting tool 30 includes an exterior case 21 having a first collection opening 22 and a collecting portion 23 movably disposed within the exterior case.

As clearly shown in Fig. 12A to Fig. 15B, the exterior case (exterior body) 21 is a spherical case having a hollow center. The first collection opening 22, which is a round opening, is formed in the side of the exterior case 21. The collecting portion 23 also has a spherical shape having a hollow center. The diameter of the collecting portion 23 is smaller than that of the exterior case 21, and the collecting portion 23 is rotatably disposed within the exterior case 21. A third collection opening 24, which is a round opening, is formed in the side of the collecting portion 23. Although the size of the first collection opening 22 is substantially the same as the size of the third collection opening 24, one of the openings may be slightly larger than the other one. However, the size of the third collection opening 24 is adjusted so that, when the collecting portion 23 rotates, the first collection opening 22 will be assuredly covered by the wall of the collecting portion 23.

One end of a cylindrical retention holder 25 is fixed to the exterior case 21, such that the retention holder 25 is perpendicular to a vertical line extending through the center of the first collection opening 22. A columnar operating stick (operating portion) 26 is rotatably provided within the retention holder 25 so as to protrude from one end of the retention holder 25.

The distal end of the operating stick 26 penetrates the exterior case 21. Inside the exterior case 21, a portion of the operating stick 26, the portion extending perpendicularly to a vertical line extending through the center of the third collection opening 24, is connected and fixed to the collecting portion 23.

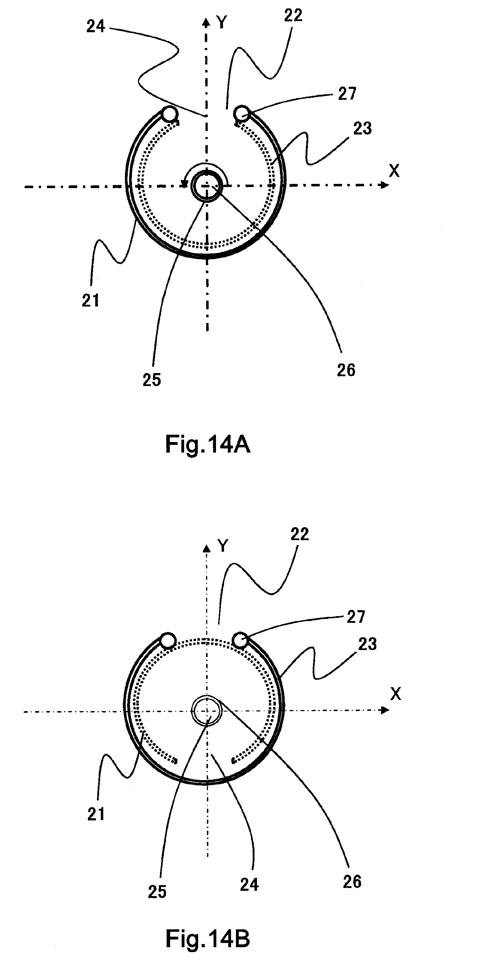

As shown in Fig. 14A to Fig. 15B, ring-shaped packing 27 having a round cross section is attached to the edge of the first collection opening 22 of the exterior case 21. The packing 27 thus attached is, at the edge of the first collection opening 22 of the exterior case 21, in contact with the outer surface of the collecting portion 23.

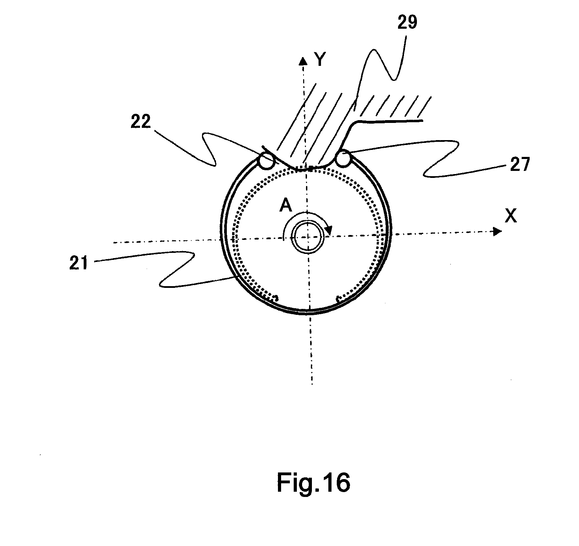

Next, a biological tissue collection method using the biological tissue collecting tool 30 according to the present embodiment is described. First, when the first collection opening 22 is in a state of being covered by the wall of the collecting portion 23, the retention holder 25 is held by finger tips, and the exterior case 21 is inserted into a nostril such that the first collection opening 22 comes into contact with the inner wall surface of the nostril. Fig. 16 shows a state where the first collection opening 22 of the exterior case 21 is in contact with the inner wall surface of the nostril. At the time, the inner wall surface of the nostril is in a state of protruding into the first collection opening 22. To be exact, since there is a mucous membrane 29 on the inner wall surface of the nostril, the mucous membrane 29 is in a state of protruding into the exterior case 21 through the first collection opening 22.

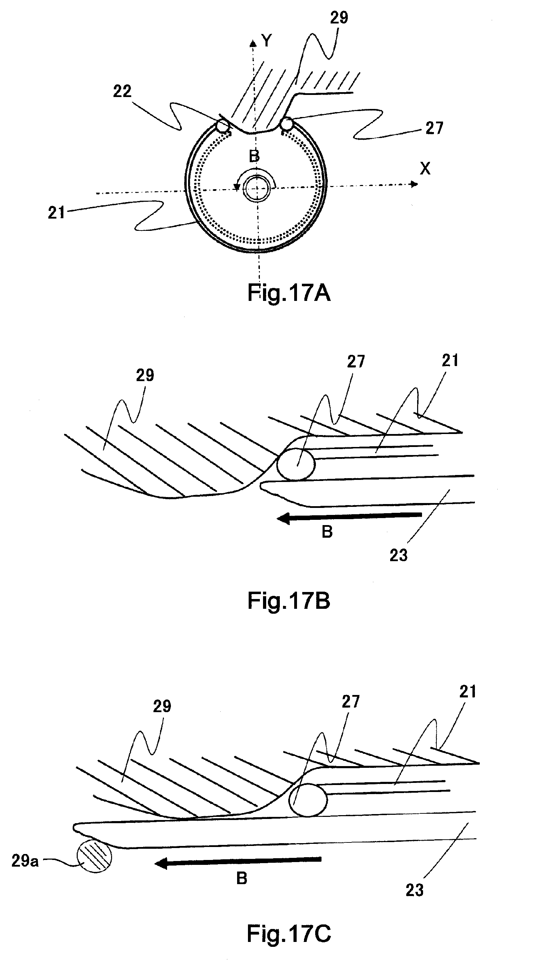

In this state, the operating stick 26 is rotated around the retention holder 25 in the direction of arrow A as shown in Fig. 16, such that the position of the first collection opening 22 and the position of the third collection opening 24 coincide with each other. As a result, as shown in Fig. 17A, the mucous membrane 29 protruding into the exterior case 21 through the first collection opening 22 is in a state of protruding into the collecting portion 23 through the third collection opening 24 of the collecting portion 23. At the time, the operating stick 26 is operated to rotate the collecting portion 23 in the direction of arrow B as shown in Fig. 17A. Consequently, the mucous membrane 29 protruding into the collecting portion 23 is scratched away by the edge of the third collection opening 24 of the collecting portion 23 as shown in Fig. 17C. Then, a mucous membrane piece 29a obtained by the scratching is stored in the inner space (i.e., a storage portion) of the collecting portion 23. Here, the edge of the third collection opening 24 of the collecting portion 23 serves as a cutting portion that cuts off the mucous membrane.

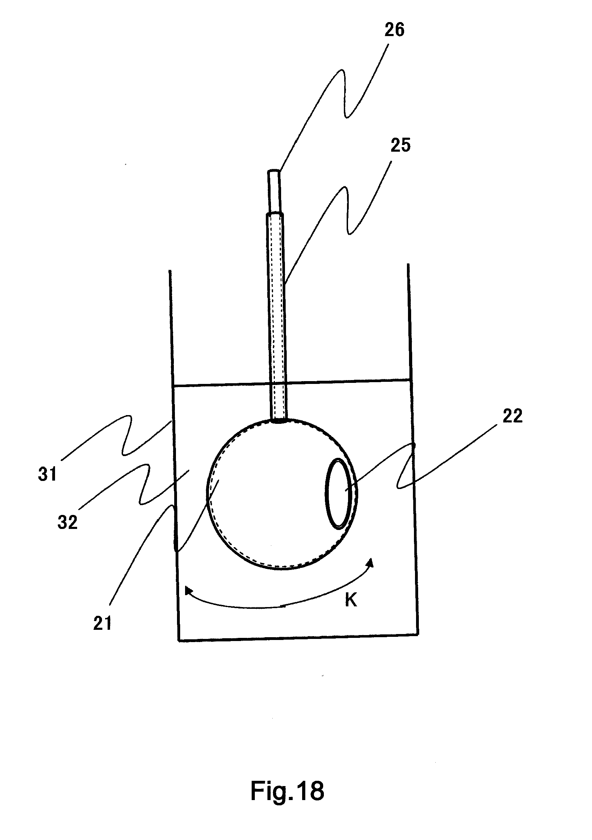

Fig. 18 illustrates an example of a method of subjecting the mucous membrane collected in the above manner to a test. A beaker 31 contains a liquid 32 for use in extracting a detection material from the mucous membrane piece 29a (examples of the liquid 32 include formic acid and a buffer solution containing formic acid). The exterior case 21 is immersed into the liquid 32, and then the operating stick 26 is operated to rotate the collecting portion 23 such that the position of the first collection opening 22 and the position of the third collection opening 24 coincide with each other, and thus the liquid 32 is flowed into the collecting portion 23. Then, the operating stick 26 is operated again to rotate the collecting portion 23 such that the first collection opening 22 is covered by the wall of the collecting portion 23. The exterior case 21 in such a state is shaken in the liquid 32 as indicated by arrow K.

As a result, a test material is extracted into the liquid 32 from the mucous membrane piece 29a stored in the collecting portion 23. Thereafter, the extraction liquid may be subjected to a measurement by a testing apparatus, and thus the amount of the test material can be measured. At the time of extraction, the first collection opening 22 of the exterior case 21 is covered by the wall of the collecting portion 23. Accordingly, the liquid 32 in the collecting portion 23 is in a predetermined volume that is determined by the volume of the inner space of the collecting portion 23. In addition, the liquid 32 in the predetermined volume contains the mucous membrane piece 29a in a predetermined amount that is determined by the area of the first collection opening 22. This makes it possible to improve the accuracy of a determination made in the test.

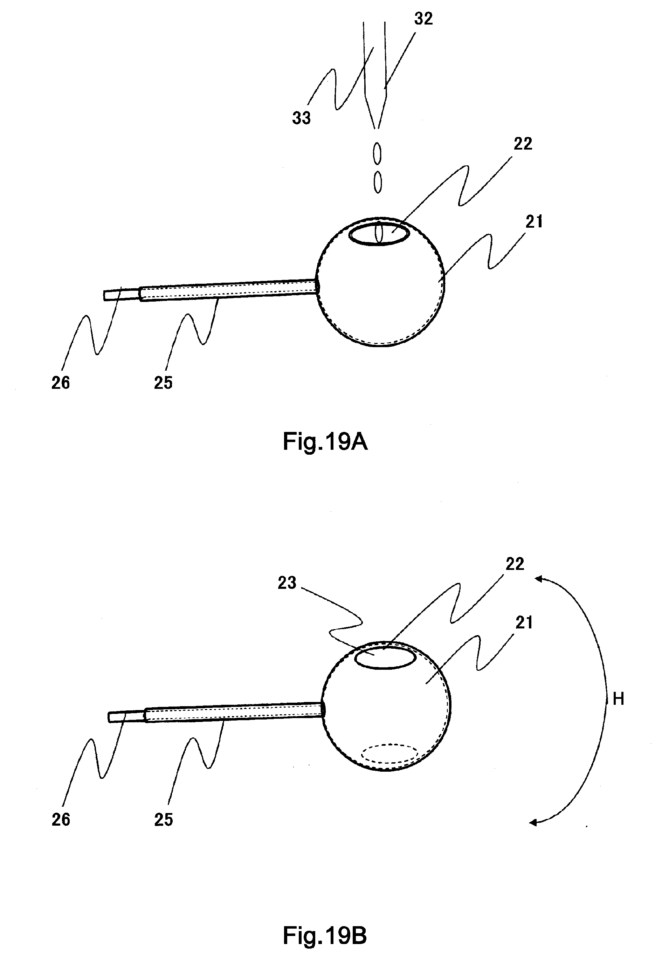

Figs. 19A and 19B illustrate another example of a method of subjecting the collected mucous membrane to a test. After the collection of the mucous membrane piece 29a into the collecting portion 23, with the first collection opening 22 of the exterior case 21 facing upward, the operating stick 26 is operated to rotate the collecting portion 23 such that the position of the first collection opening 22 and the position of the third collection opening 24 coincide with each other. Then, as shown in Fig. 19A, a dropper 33 is used to flow the liquid 32 into the collecting portion 23 through the first collection opening 22 and the third collection opening 24. Next, as shown in Fig. 19B, with the first collection opening 22 kept facing upward, the operating stick 26 is operated to rotate the collecting portion 23 such that the first collection opening 22 is covered by the wall of the collecting portion 23. In this state, the exterior case 21 is shaken in the direction of arrow H. As a result, inside the collecting portion 23, a test material is extracted from the mucous membrane piece 29a into the liquid 32. Thereafter, the extraction liquid may be subjected to a measurement by a testing apparatus, and thus the amount of the test material can be measured.

The above description gives an example where the exterior case 21 and the collecting portion 23 are spherical. However, as an alternative, the exterior case 21 and the collecting portion 23 may be oval-spherical components, each of which has a long axis extending in the axial direction of the operating stick 26. For example, each of the oval-spherical exterior case and the oval-spherical collecting portion may have a long axis longer than the diameter of the spherical exterior case, and may have a short axis whose length is the same as the diameter of the spherical exterior case. In this manner, the length of each of the exterior case and the collecting portion in the direction of the short axis perpendicular to the operating stick 26 can be made relatively short without reducing the area of the collection opening. This makes it possible to smoothly insert the exterior case into a biological aperture even if the biological aperture into which the exterior case is to be inserted is small.

### (Embodiment 4)

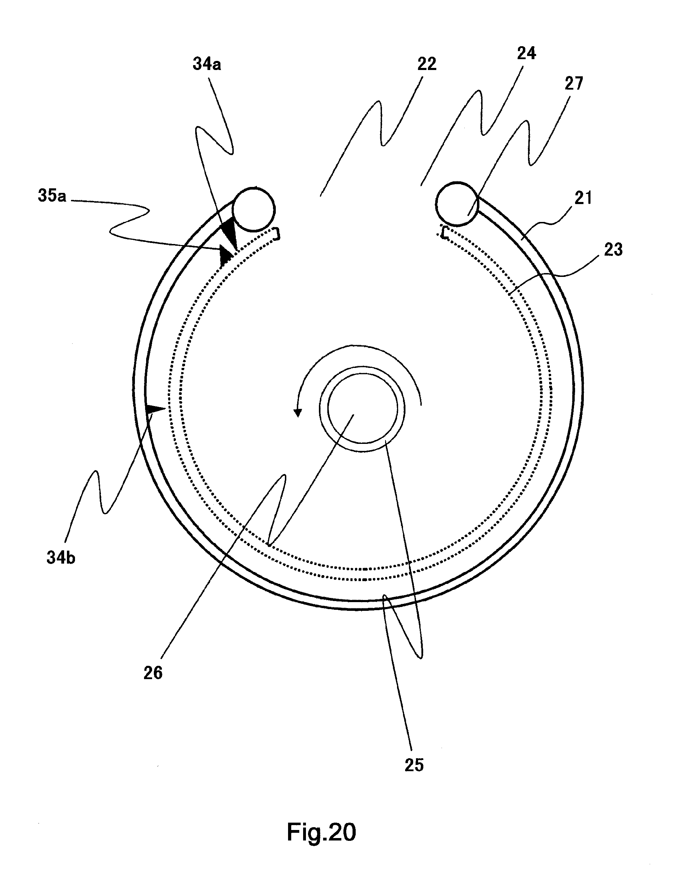

Fig. 20 shows a biological tissue collecting tool 40 according to Embodiment 4. Hereinafter, only differences of the present embodiment from Embodiment 3 are described. In the present embodiment, protrusion-like rotation restriction portions 34a and 34b are provided on the inner wall surface of the exterior case 21, and a protrusion-like rotation restriction portion 35a is provided on the outer wall surface of the collecting portion 23 so as to be positioned between the rotation restriction portions 34a and 34b.

Accordingly, the rotation of the collecting portion 23 is restricted within a range that is defined by a movement range of the rotation restriction portion 35a, over which movement range the rotation restriction portion 35a is movable without coming into contact with the rotation restriction portion 34a or 34b of the exterior case 21. In this manner, the rotation of the collecting portion 23 can be restricted within a necessary range. It should be noted that the positions of the rotation restriction portions 34a and 34b and the rotation restriction portion 35a are set so that the first collection opening 22 of the exterior case 21 can be opened and closed through the aforementioned movement of the collecting portion 23.

### (Embodiment 5)

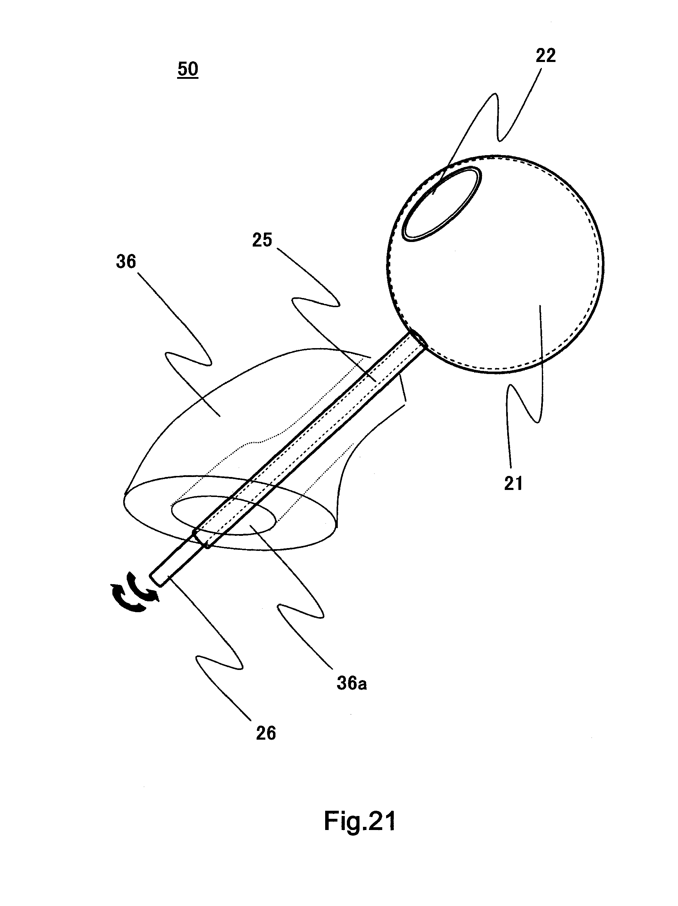

Fig. 21 is a perspective view of a biological tissue collecting tool 50 according to Embodiment 5. The biological tissue collecting tool 50 is configured such that an insertion assisting tool 36 is attached to the outer periphery of the cylindrical retention holder 25 of the biological tissue collecting tool 30 according to Embodiment 3. The insertion assisting tool 36 has a shape that is fitted to the internal shape of a nostril. Inside the insertion assisting tool 36, space allowing the retention holder 25 to be inserted therein is formed as a cylindrical guide portion 36a. By inserting the insertion assisting tool 36 into a nostril together with the exterior case 21, the retention holder 25 can be stably inserted in a predetermined part of the inside of the nostril.

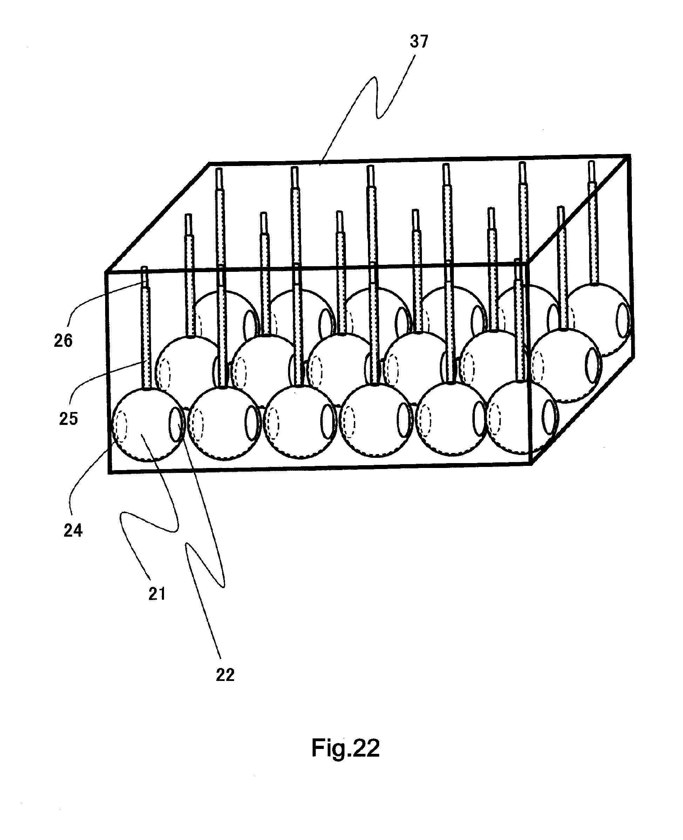

Fig. 22 is a perspective view showing a state where a plurality of biological tissue collecting tools 30 according to Embodiment 3 are arranged for storage. In a storage case 37, a plurality of unused biological tissue collecting tools 30 are arranged for storage. At the time, each biological tissue collecting tool 30 is in a state where the position of the first collection opening 22 of the exterior case 21 and the position of the third collection opening 24 of the collecting portion 23 are not coincident with each other. In this manner, dusts or the like are prevented from entering the collecting portion 23 through the first collection opening 22.

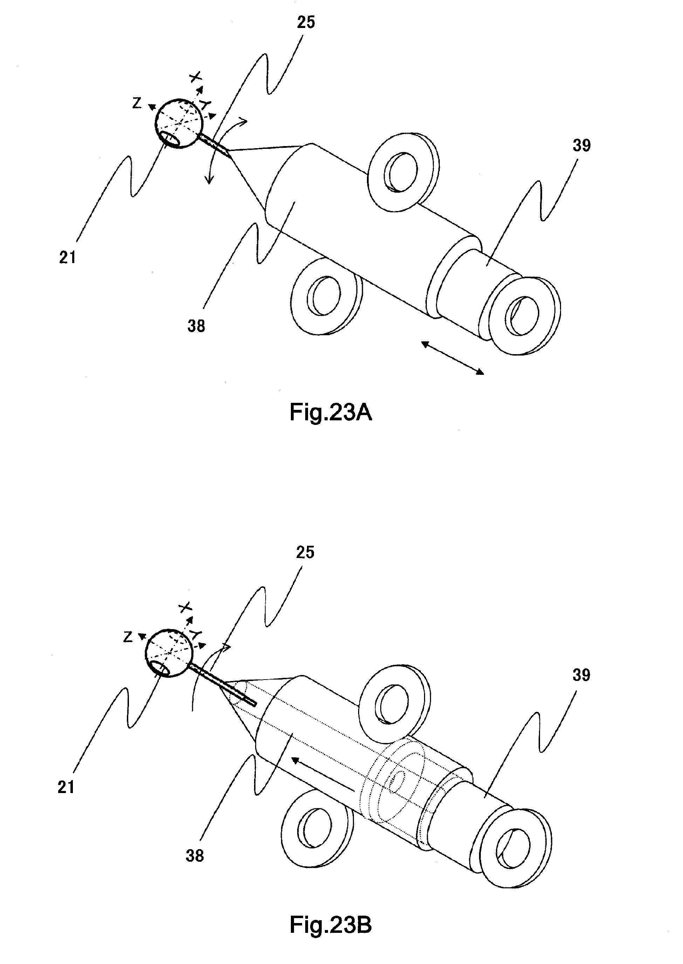

Figs. 23A and 23B are perspective views, each showing the biological tissue collecting tool 30 according to Embodiment 3 in combination with an operating tool. The operating tool includes an external cylinder 38 and an internal cylinder 39. The internal cylinder 39 is configured such that the front end side of the internal cylinder 39 is inserted in the external cylinder 38 and the rear end side of the internal cylinder 39 protrudes to the outside of the external cylinder 38. The internal cylinder 39 is inserted in the external cylinder 38 such that the internal cylinder 39 is movable in the front-rear direction as well as the circumferential direction. A retainer (not shown) configured to hold the operating stick 26 of the biological tissue collecting tool 30 is provided at the front end side of the internal cylinder 39. Similarly, a retainer (not shown) configured to hold the retention holder 25 is provided at the front end side of the external cylinder 38.

Accordingly, as shown in Fig. 23A, the retention holder 25 and the operating stick 26 of the biological tissue collecting tool 30 are held by the external cylinder 38 and the internal cylinder 39 of the operating tool, respectively. In such a state, the exterior case 21 is inserted into a nostril. At the time, the internal cylinder 39 is rotated while the external cylinder 38 is fixedly held. As a result, the third collection opening 24 of the collecting portion 23 moves to scratch away the mucous membrane 29, and the mucous membrane piece 29a obtained by the scratching can be stored in the collecting portion 23.

### (Embodiment 6)

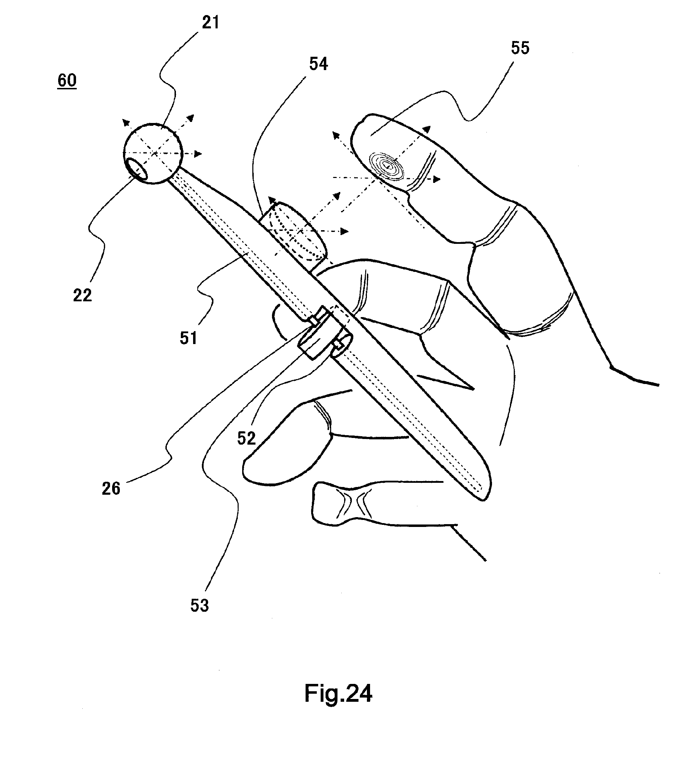

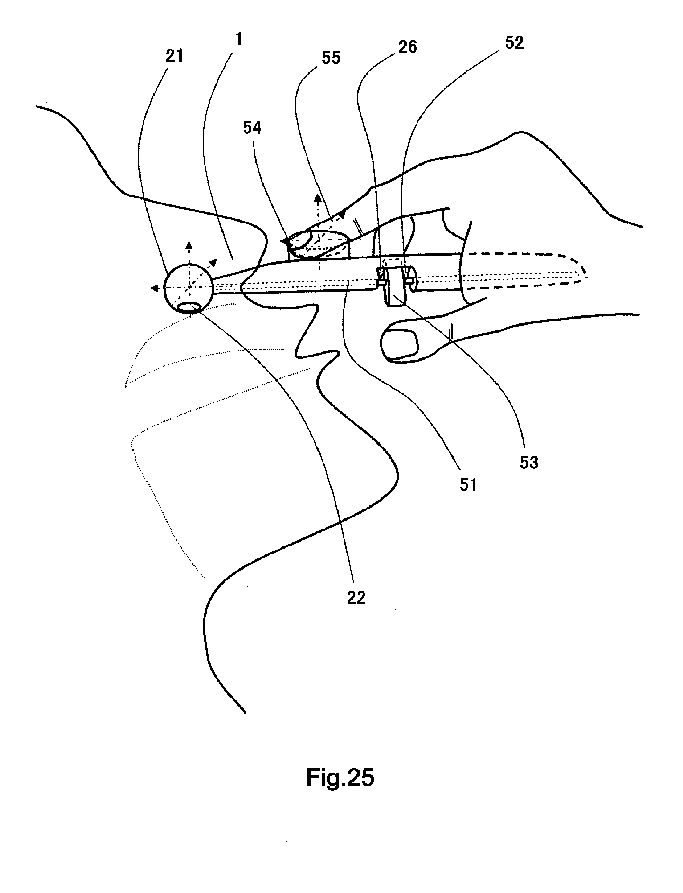

Figs. 24 to 26B show a biological tissue collecting tool 60 according to Embodiment 6. Hereinafter, differences of the biological tissue collecting tool 60 from the biological tissue collecting tool 30 according to Embodiment 3 are described.

A retention holder 51, which has a hollow center therein, is fixed to the exterior case 21 so as to be perpendicular to the vertical line extending through the center of the first collection opening 22. The columnar operating stick 26 is rotatably provided within the retention holder 51.

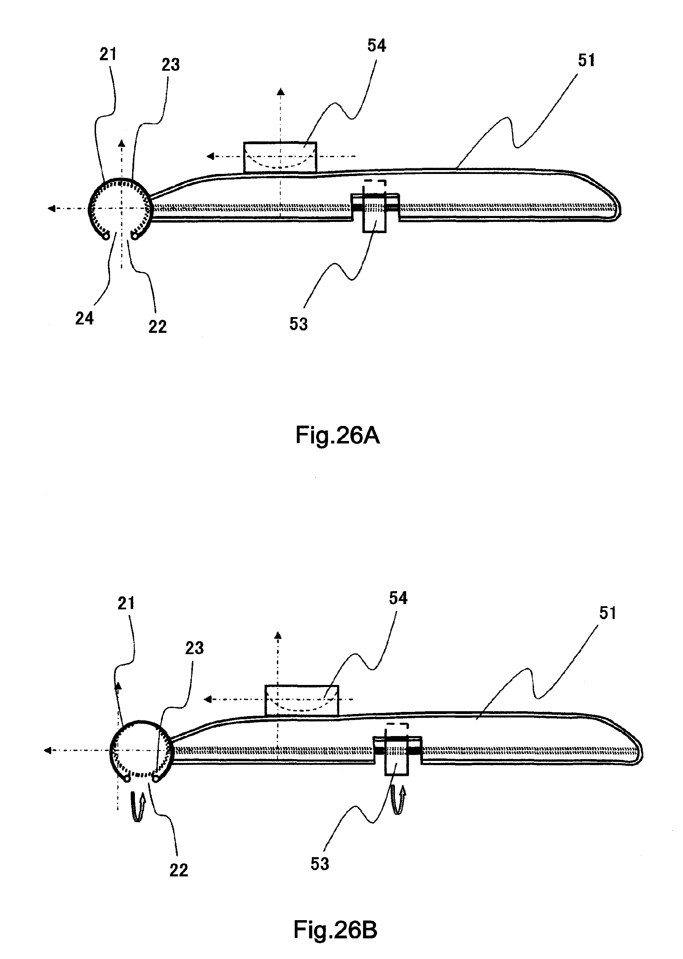

A notch 52 is formed in the retention holder 51 at a position corresponding to a middle portion of the operating stick 26, such that the middle portion of the operating stick 26 is exposed from the retention holder 51 at the same side as the first collection opening 22. At the notch 52, a disc-shaped operating portion 53 is fixedly connected to the exposed operating stick 26. As shown in Figs. 26A and 26B, the collecting portion 23 can be rotated within the exterior case 21 by rotating the operating portion 53 perpendicularly to the operating stick 26.

A finger rest 54 is provided at the opposite side to the first collection opening 22 and the notch 52. The finger rest 54 is provided between the exterior case 21 and a position where the notch 52 and the operating portion 53 are provided.

In a case where the biological tissue collecting tool 70 is used, the retention holder 51 is held by one hand with an index finger 55 placed on the finger rest 54. By holding the retention holder 51 in such a manner, the operating portion 53 can be rotated with a thumb. Thus, with use of only one hand, the biological tissue collecting tool 70 can be held and the operating stick 26 and the collecting portion 23 can be readily rotated.

In the present embodiment, the finger rest 54 on which the index finger 55 can be placed is provided at a middle portion of the retention holder 51 at the opposite side to the first collection opening 22. Therefore, the first collection opening 22 of the exterior case 21 can be properly brought into contact with the mucous membrane 29 to be collected. This makes it possible to further stabilize the amount of biological tissue to be collected each time the tissue collection is performed, and thereby to improve the accuracy of a determination made in a test. Further, at the time of inserting the exterior case 21 into a nostril, the position of the first collection opening 22 of the exterior case 21 in the nostril can be estimated based on a positional relationship between the nostril and the finger rest 54. This enhances operability and usability.

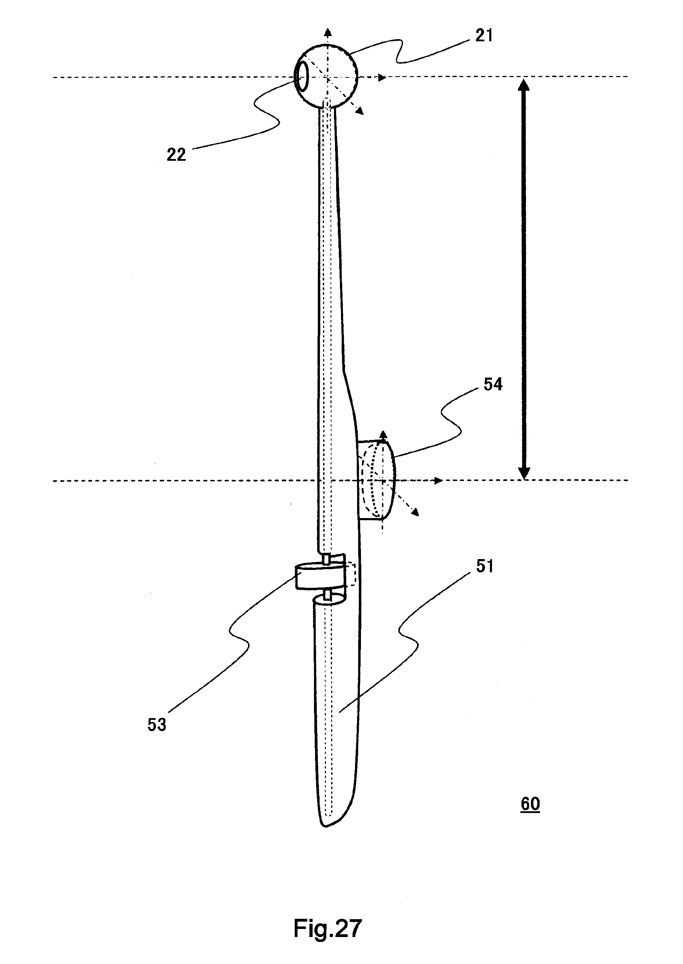

Fig. 27 shows the biological tissue collecting tool 60 according to the present embodiment in another mode. In this mode, the length from the exterior case 21 to the finger rest 54 is set to be longer than that of the biological tissue collecting tool 60 shown in Fig. 24. By preparing a plurality of biological tissue collecting tools 60 that are different from each other in terms of the length from the exterior case 21 to the finger rest 54, differences among persons in terms of the length of the common nasal meatus can be accommodated.

In order to vary the length from the exterior case 21 to the finger rest 54, the front side portion of the biological tissue collecting tool 60 up to the finger rest 54 (i.e., a portion close to the exterior case 21) may be formed as a detachable portion, and a plurality of such detachable front side portions having different respective lengths may be prepared.

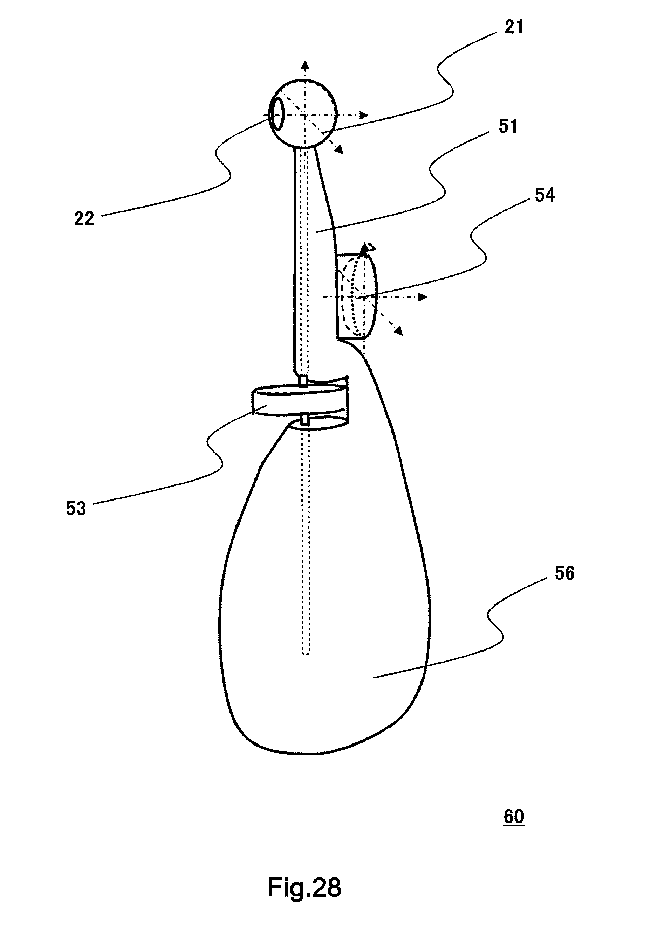

Fig. 28 shows the biological tissue collecting tool 70 according to the present embodiment in yet another mode. In this mode, the diameter of a portion of the retention holder 51, the portion being positioned at the rear of the finger rest 54, is increased and thereby a grip 56 is formed, which facilitates the holding of the retention holder 51.

### (Embodiment 7)

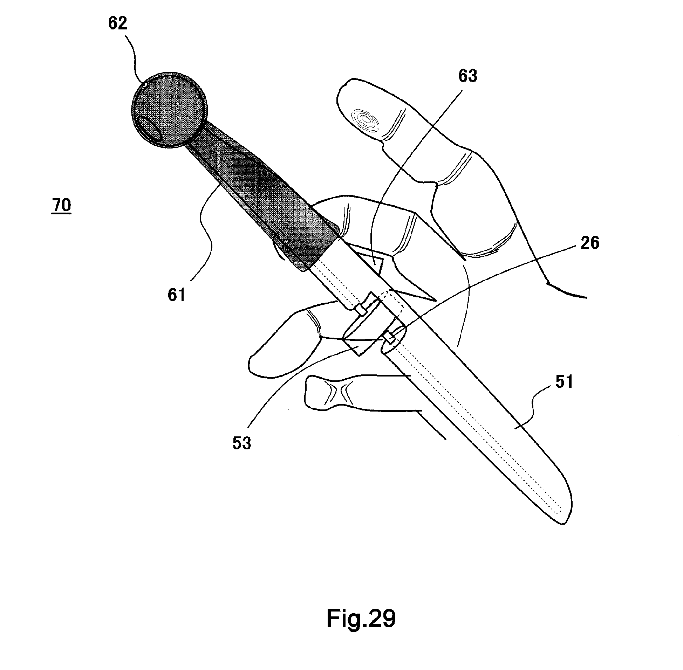

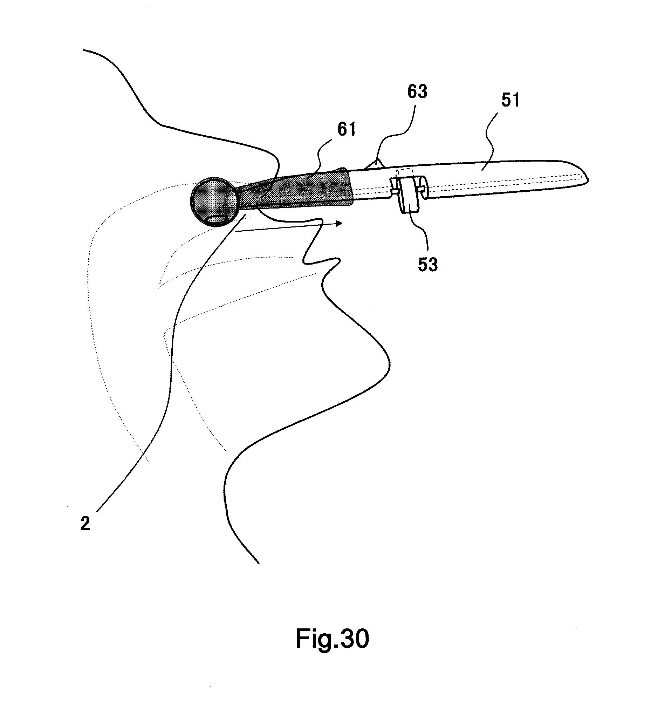

Figs. 29 and 30 show a biological tissue collecting tool 70 according to Embodiment 7. Hereinafter, differences of the biological tissue collecting tool 70 from the biological tissue collecting tool 60 according to Embodiment 6 are described.

The exterior case 21 and the outer periphery of the distal end side of the retention holder 51 of the biological tissue collecting tool 70 are covered by a pouch-shaped wipe cover 61 which has a closed front end and an open rear end.

The wipe cover 61 has the following shape: the front end portion has a spherical shape conforming to the shape of the outer periphery of the exterior case 21; and the remaining portion has a cylindrical shape whose diameter increases toward the rear end. The wipe cover 61 is formed of a net-like synthetic resin fiber or plant fiber. The wipe cover 61 is hydrophilic and has an irregular surface. By using the wipe cover 61, the inside of a nostril can be cleaned.

A tearing beginning point 62 is formed at the distal end of the wipe cover 61. The tearing beginning point is a point where the wipe cover 61 begins to be torn apart when pulled toward the rear end of the retention holder 51. For example, the tearing beginning point 62 may be formed by forming a small opening at the distal end of the wipe cover 61, or by forming a cut in the distal end of the wipe cover 61, or by forming a thin thickness portion at the distal end of the wipe cover 61.

A diameter expanding portion 63 is provided at the opposite side to the first collection opening 22 and the notch 52. The diameter expanding portion 63 is provided between the exterior case 21 and a position where the notch 52 and the operating portion 53 are provided. The diameter of a portion of the retention holder 51 where the diameter expanding portion 63 is provided is greater than the diameter of a portion of the retention holder 51 that is closer to the distal end than the portion where the diameter expanding portion 63 is provided.

At the time of using the biological tissue collecting tool 70, first, the wipe cover 61 is attached so as to cover the exterior case 21 and the distal end side of the retention holder 51 of the biological tissue collecting tool 70. At the time, the rear end of the wipe cover 61 is positioned closer to the distal end of the retention holder 51 than the diameter expanding portion 63. In such a state, the exterior case 21 covered by the wipe cover 61 is inserted into a nostril. Next, the entire biological tissue collecting tool 70 is rotated or moved back and forth within the nostril to clean the inside of the nostril. That is, materials existing inside the nostril that are unnecessary for a test are removed in advance.

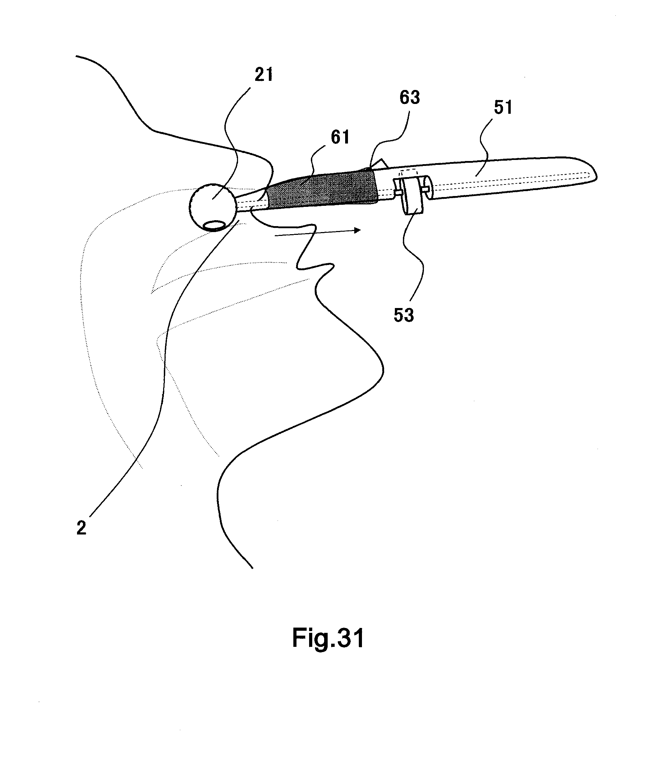

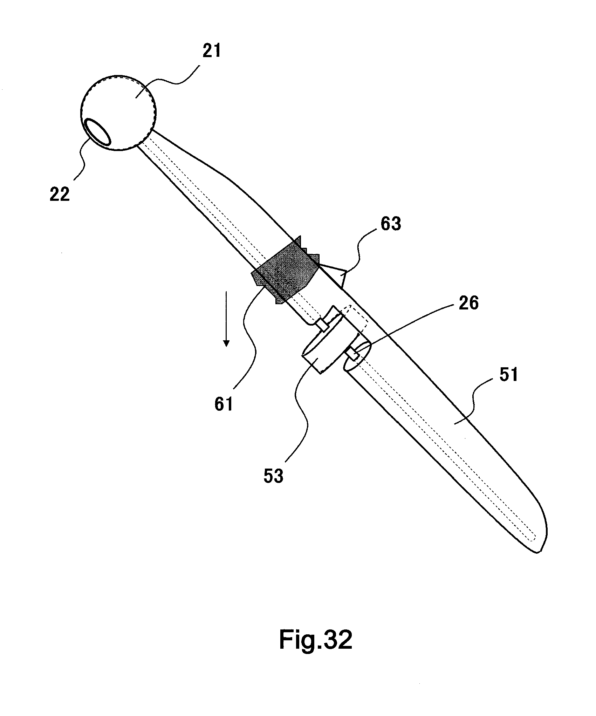

After cleaning the inside of the nostril, the rear end of the wipe cover 61 is held and pulled toward the rear end of the biological tissue collecting tool 70 (toward the outside of the nostril). As a result, the tearing beginning point 62 at the distal end of the wipe cover 61 is torn apart first, and then the tear becomes larger as the wipe cover 61 is pulled toward the rear end of the biological tissue collecting tool 70. Consequently, as shown in Fig. 31 and Fig. 32, the wipe cover 61 is moved to be positioned at the rear of the exterior case 21.

By pulling the wipe cover 61 toward the rear end of the biological tissue collecting tool 70 in the above manner, the exterior case 21 is exposed inside the nostril. Thereafter, the first collection opening 22 of the exterior case 21 is brought into contact with the inner wall surface of the nostril and the collecting portion 23 is rotated. In this manner, the mucous membrane 29 can be scratched away.

As described above, in the present embodiment, the inside of the nostril can be cleaned through the wiping with the wipe cover 61 before the mucous membrane 29 is scratched away. Therefore, collection of unnecessary materials from the inside of the nostril is reduced in addition to that the amount of biological tissue to be collected each time the tissue collection is performed is stabilized. This makes it possible to further improve the accuracy of a determination made in a test.

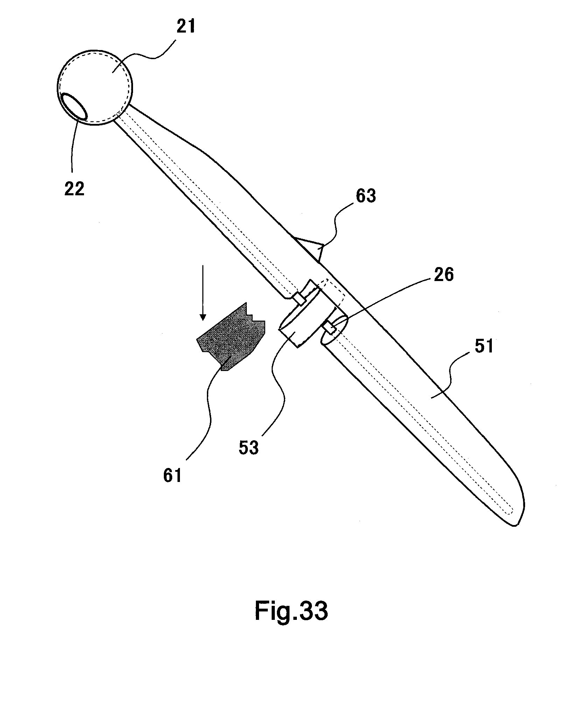

Before the biological tissue collected by the biological tissue collecting tool 70 is subjected to a test, the wipe cover 61 can be removed from the biological tissue collecting tool 70 as shown in Fig. 33.

Specifically, when the rear end of the wipe cover 61 comes into contact with the diameter expanding portion 63 as shown in Fig. 32 as a result of the wipe cover 61 being pulled toward the rear end of the biological tissue collecting tool 70, the tearing of the wipe cover 61 progresses from the rear end toward the front end of the wipe cover 61 at the diameter expanding portion 63. Consequently, as shown in Fig. 33, the torn wipe cover 61 can be readily removed from the biological tissue collecting tool.

### (Embodiment 8)

A biological tissue collecting tool 80 according to Embodiment 8 is the same as the biological tissue collecting tool 60 according to Embodiment 6 except that the collecting portion of the tool 80 is different from the collecting portion of the tool 60. Hereinafter, differences of the biological tissue collecting tool 80 from the biological tissue collecting tool 60 are described.

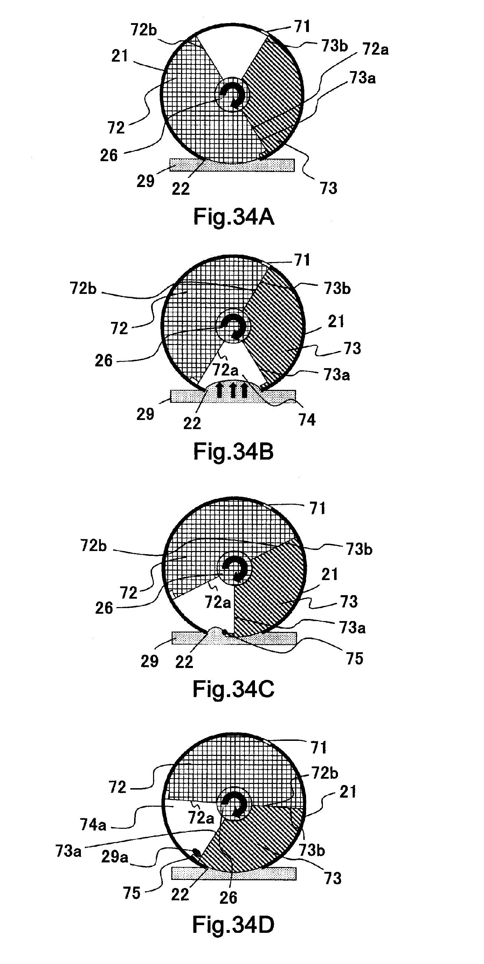

Figs. 34A and 34B are cross-sectional views, each showing a cross section of the exterior case 21 and the collecting portion of the biological tissue collecting tool 80 taken along a line perpendicular to the operating stick 26.

The collecting portion of the biological tissue collecting tool 80 includes a first rotating piece 72 and a second rotating piece 73 which are rotatably arranged along the inner wall of the exterior case 21. As shown in Figs. 34A and 34B, the first rotating piece 72 has a hemispherical shape (of 180°) which is obtained by dividing a sphere having a hollow center into half, and the second rotating piece 73 has a shape (of 120°) which is obtained by dividing a sphere having a hollow center into three equal parts.

The distal end of the operating stick 26 is fixed to the first rotating piece 72. Similar to Embodiment 6, the operating stick 26 is provided with the disc-shaped operating portion 53. Therefore, by rotating the operating portion 53 with a thumb, the first rotating piece 72 can be rotated within the exterior case 21 as shown in Figs. 34A to 34D. It should be noted that the second rotating piece 73 is not connected to the operating stick 26.

In Fig. 34A, the first collection opening 22 is covered by the spherical outer peripheral surface of the first rotating piece 72, and the second rotating piece 73 is disposed at such a position as not to cover the first collection opening 22. At the time, a first edge 72a of the first rotating piece 72 and a first edge 73a of the second rotating piece 73 are in contact with each other. In contrast, a second edge 72b of the first rotating piece 72 and a second edge 73b of the second rotating piece 73 are spaced apart from each other. At the time of inserting the exterior case 21 into a nostril, the first rotating piece 72 and the second rotating piece 73 are arranged so as to be in such as state as shown in Fig. 34A. In Fig. 34A, the first collection opening 22 is in contact with the mucous membrane 29.

It should be noted that an air hole 71, which is a through-hole, is formed in the outer wall of the exterior case 21. The air hole 71 is provided at such a position as to face an area sandwiched between the second edge 72b of the first rotating piece 72 and the second edge 73b of the second rotating piece 73.

Next, when the operating portion 53 is rotated clockwise in Fig. 34A, the first rotating piece 72 rotates, accordingly. As a result, as shown in Fig. 34B, the second edge 72b of the first rotating piece 72 and the second edge 73b of the second rotating piece 73 come into contact with each other. In contrast, the first edge 72a of the first rotating piece 72 and the first edge 73a of the second rotating piece 73 become spaced apart from each other, so that the first collection opening 22 is opened inside the nostril. During the transition from the state shown in Fig. 34A to the state shown in Fig. 34B, space 74 is formed between the first edge 72a of the first rotating piece 72 and the first edge 73a of the second rotating piece 73, and the space is under negative pressure.

Consequently, as shown in Fig. 34B, a part of the mucous membrane 29 in the nostril protrudes into the exterior case 21 through the first collection opening 22.

Air existing in the space sandwiched between the second edge 72b of the first rotating piece 72 and the second edge 73b of the second rotating piece 73 in Fig. 34A is released to the outside of the exterior case 21 through the air hole 71 during the transition from the state shown in Fig. 34A to the state shown in Fig. 34B.

In the state shown in Fig. 34B, when the operating portion 53 is further rotated clockwise in Fig. 34B, the first rotating piece 72 rotates accordingly, and also, the second rotating piece 73 rotates clockwise since the second edge 73b of the second rotating piece 73 is pushed by the second edge 72b of the first rotating piece 72. As a result, the second edge 73b of the second rotating piece 73 passes through the first collection opening 22, and a scratching portion 75 provided at the outer periphery of the first edge 73a of the second rotating piece 73 scratches away the mucous membrane 29 protruding into the first collection opening 22 as shown in Fig. 34C.

Subsequently, in the state shown in Fig. 34C, when the operating portion 53 is further rotated clockwise in Fig. 34C, the first collection opening 22 becomes covered by the spherical outer peripheral surface of the second rotating piece 73 as shown in Fig. 34D. At the time, the mucous membrane 29 obtained by the scratching is stored in storage space 74a which is space formed between the first edge 72a of the first rotating piece 72 and the first edge 73a of the second rotating piece 73. In this state, the exterior case 21 is pulled out of the nostril.

It should be noted that the air hole 71, which is formed in an area different from the first collection opening 22 of the exterior case 21, is preferably provided at such a position as not to face the storage space 74a, because if the air hole 71 faces the storage space 74a, then the mucous membrane piece 29a obtained by the scratching is released to the outside of the exterior case 21 through the air hole 71.

### (Embodiment 9)

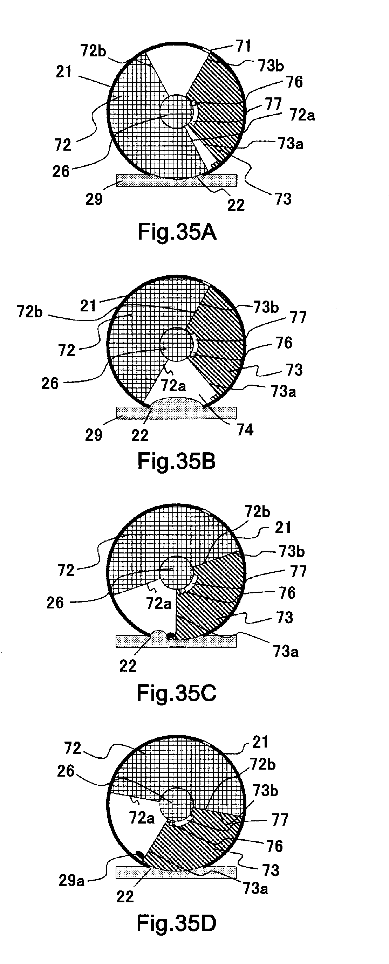

Figs. 35A and 35B are cross-sectional views, each showing a cross section of the exterior case 21 and the collecting portion of a biological tissue collecting tool 90 according to Embodiment 9 taken along a line perpendicular to the operating stick 26. The biological tissue collecting tool 90 according to the present embodiment is similar to the biological tissue collecting tool 80 according to Embodiment 8. Hereinafter, differences of the biological tissue collecting tool 90 from the biological tissue collecting tool 80 are described.

The collecting portion of the biological tissue collecting tool 90 includes a contact preventer configured to prevent the first edge 72a of the first rotating piece 72 and the first edge 73a of the second rotating piece 73 from coming into contact with each other. The contact preventer is configured such that a protrusion 76 provided at the operating stick 26 is engaged with a groove 77 formed in a portion of the second rotating piece 73, the portion facing the operating stick 26. The groove 77 is a recess having a width that is greater than the width of the protrusion 76.

In Fig. 35A, similar to Fig. 34A, the first collection opening 22 is covered by the spherical outer peripheral surface of the first rotating piece 72. However, due to the presence of the contact preventer, the first edge 72a of the first rotating piece 72 and the first edge 73a of the second rotating piece 73 are not in contact with each other. At the time, the protrusion 76 is positioned at a side edge of the groove 77 at the second edge 73b side. In this state, the exterior case 21 is inserted into a nostril.

Next, the operating portion 53 is rotated clockwise in Fig. 35A. Consequently, as shown in Fig. 35B, space 74 is formed between the first edge 72a of the first rotating piece 72 and the first edge 73a of the second rotating piece 73, and the second edge 72b of the first rotating piece 72 and the second edge 73b of the second rotating piece 73 come into contact with each other. At the time, the protrusion 76 is positioned at a side edge of the groove 77 at the first edge 73a side. Such a position of the protrusion 76 in the groove 77 is maintained as shown in Figs. 35C and 35D until the mucous membrane 29 is collected.

In the state shown in Fig. 34D or Fig. 35D, in order to retrieve the collected mucous membrane 29 from the exterior case 21, the operating portion 53 is further rotated clockwise such that the space 74 faces the first collection opening 22. In such a state, the mucous membrane piece 29a may be caused to flow into, for example, pure water.

In the biological tissue collecting tool 80 according to Embodiment 8, if the operating stick 26 is rotated counterclockwise at the time of retrieving the mucous membrane piece 29a from the exterior case 21, then there is a risk that the first edge 72a of the first rotating piece 72 and the first edge 73a of the second rotating piece 73 come into contact with each other, which may result in that the collected mucous membrane piece 29a becomes compressed. However, in the biological tissue collecting tool 90 provided with the contact preventer, such compression of the collected mucous membrane piece 29a can be avoided since the first edge 72a of the first rotating piece 72 and the first edge 73a of the second rotating piece 73 are prevented from coming into contact with each other. To be specific, in the biological tissue collecting tool 90, if the operating stick 26 in the state shown in Fig. 35D is erroneously rotated counterclockwise, then the protrusion 76 first comes into contact with the side edge of the groove 77 at the second edge 73b side. Thereafter, in such a state, the second rotating piece 73 is rotated. Therefore, there is not a risk that the first edge 72a of the first rotating piece 72 and the first edge 73a of the second rotating piece 73 come into contact with each other.

### (Embodiment 10)

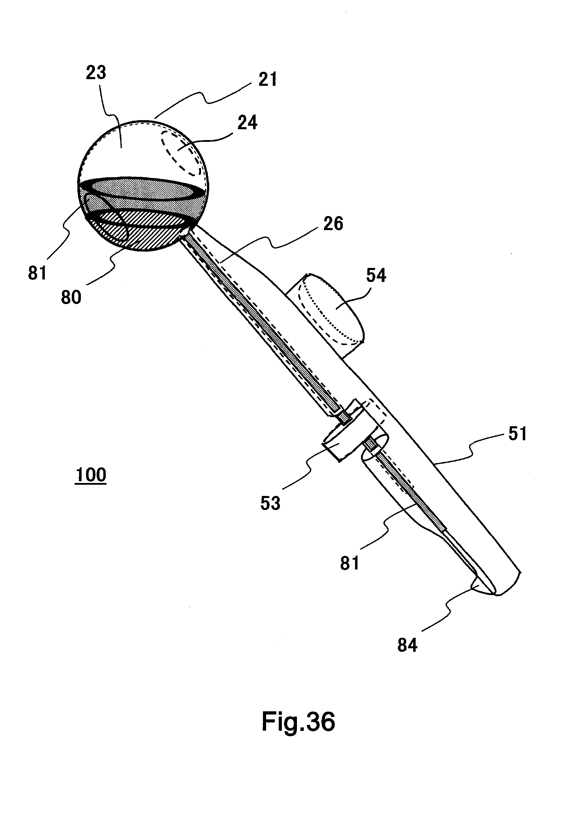

Fig. 36 shows a biological tissue collecting tool 100 according to Embodiment 10. Hereinafter, differences of the biological tissue collecting tool 100 from the biological tissue collecting tool 60 according to Embodiment 6 are described.

In the biological tissue collecting tool 100, the operating stick 26 is formed of a cylindrical member having a hollow center. The cylindrical operating stick 26 accommodates a columnar reagent body 81 therein, such that the reagent body 81 is movable within the operating stick 26 in the axial direction. The reagent body is a columnar member formed of a water-absorbing fiber. The reagent body is pre-impregnated with a reagent, the reagent changing its color when reacting with a test object. A push-in operating portion 84 is connected to one end of the reagent body 81 (at the opposite side to the exterior case 21). With the use of the push-in operating portion 84, the reagent body 81 can be moved within the operating stick 26 toward the exterior case 21 and the collecting portion 23.

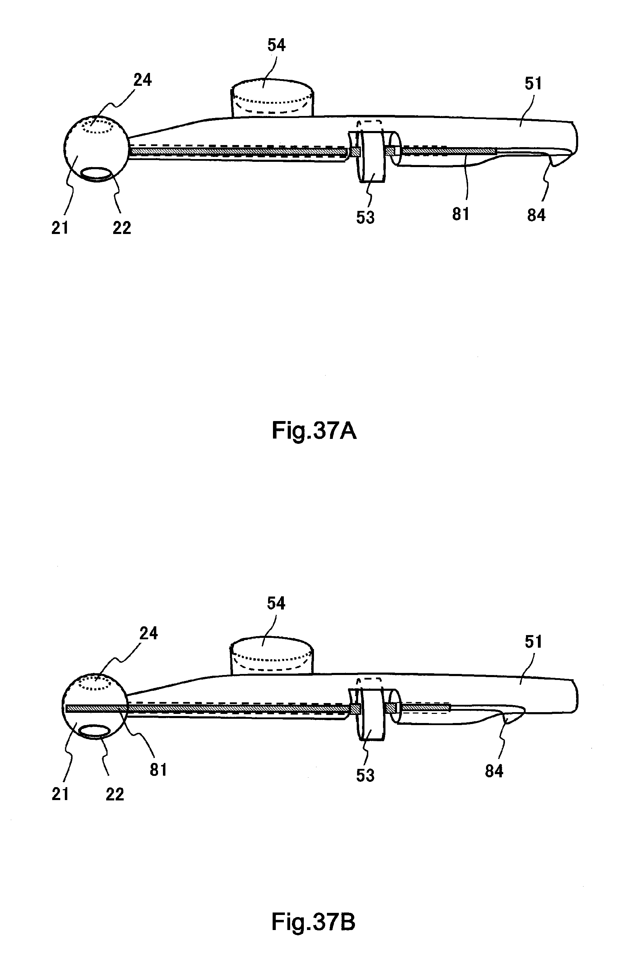

At a position where the distal end of the reagent body 81 faces the outer wall surface of the collecting portion 23, the outer wall surface is formed as a rupture membrane (not shown). Accordingly, when the reagent body 81 is moved toward the exterior case 21 and the collecting portion 23 as shown in Figs. 37A and 37B, the distal end of the reagent body 81 passes through the rupture membrane and is inserted into the collecting portion 23.

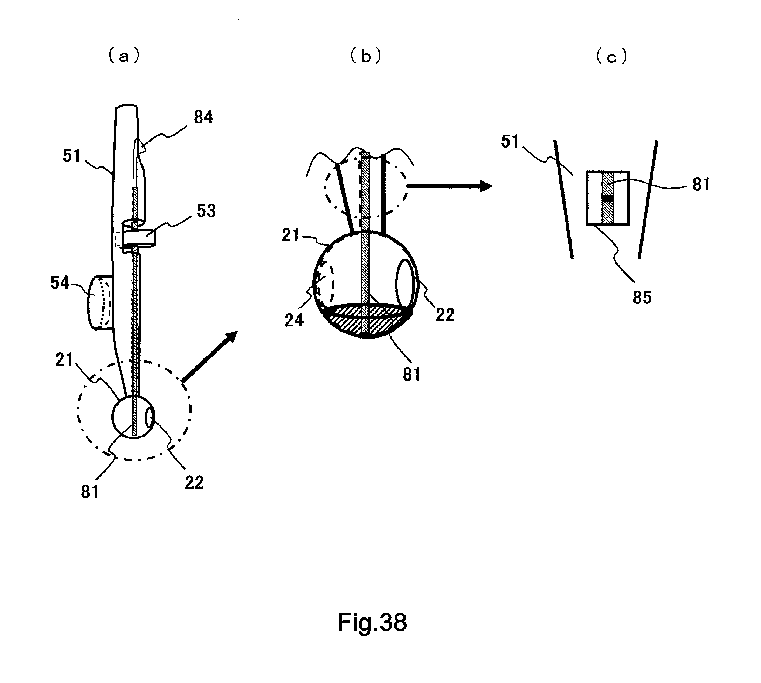

As shown in Fig 38, the wall of the retention holder 51 is partially eliminated at a position between the exterior case 21 and the operating portion 53, and a reaction confirmation window 85 is provided in the wall at the position. Through the reaction confirmation window 85, the state of the reagent body 81 can be confirmed. In order to facilitate the confirmation, the operating stick 26 is formed of a transparent material.

A gel-like neutralizer 86, which melts when heated, is disposed within the collecting portion 23. Further, a gel-like reaction body 87, which melts when heated, is disposed on top of the neutralizer 86. The neutralizer herein includes, for example, formic acid, and the reaction body herein includes, for example, an alkali that neutralizes formic acid.

Described next is a test method in which: the biological tissue collecting tool 100 according to the present embodiment is used to collect a mucous membrane from a nostril; and then whether amyloid beta exists in the mucous membrane, or how much amount of amyloid beta exists in the mucous membrane, is determined.

First, a mucous membrane is collected from a nostril by using the biological tissue collecting tool 100 in a manner similar to the usage of the biological tissue collecting tool 60 according to Embodiment 6. Fig. 37A shows a state of the biological tissue collecting tool 100 when the collection of the mucous membrane is completed.

In this state, heat is applied to the biological tissue collecting tool 100 by means of, for example, a heater from above the exterior case 21 in Fig. 36. As a result, first, the reaction body 87 disposed at an upper position in the collecting portion 23 melts into a liquid. Accordingly, amyloid beta that may exist in the mucous membrane collected by the collecting portion 23 reacts with the reaction body 87 and is decomposed, so that the amyloid beta transforms from an aggregate into simple substances.

When the heating by the heater is further continued, the neutralizer 86 disposed at a lower position in the collecting portion 23 melts into a liquid. As a result, the reaction body 87 is neutralized.

Next, as shown in Fig. 37B, the push-in operating portion 84 is held to move the reagent body 81 toward the exterior case 21 and the collecting portion 23, so that the distal end of the reagent body 81 protrudes into the collecting portion 23 by tearing through the rupture membrane. As a result, the distal end of the reagent body 81 is immersed into the liquid in the collecting portion 23. Since the reagent body 81 is pre-impregnated with a reagent that reacts with amyloid beta, the reagent body 81 changes its color if amyloid beta exists in the collected mucous membrane. Since the reagent body 81 is formed of a water-absorbing fiber, the liquid in the collecting portion 23 is absorbed by the fiber due to a capillary phenomenon and rises through the reagent body 81. Consequently, the state of the reaction of the reagent can be observed through the reaction confirmation window 85.

According to the present embodiment, from when the mucous membrane is collected to when the reaction of the reagent occurs, the collected mucous membrane is kept inside the biological tissue collecting tool 100, and thus hygienic quality can be improved.

### (Embodiment 11)

Hereinafter, differences of a biological tissue collecting tool 110 according to Embodiment 11 from the biological tissue collecting tool 100 according to Embodiment 10 are described.

In the biological tissue collecting tool 110, neither the neutralizer 86 nor the reaction body 87 is disposed within the collecting portion 23.

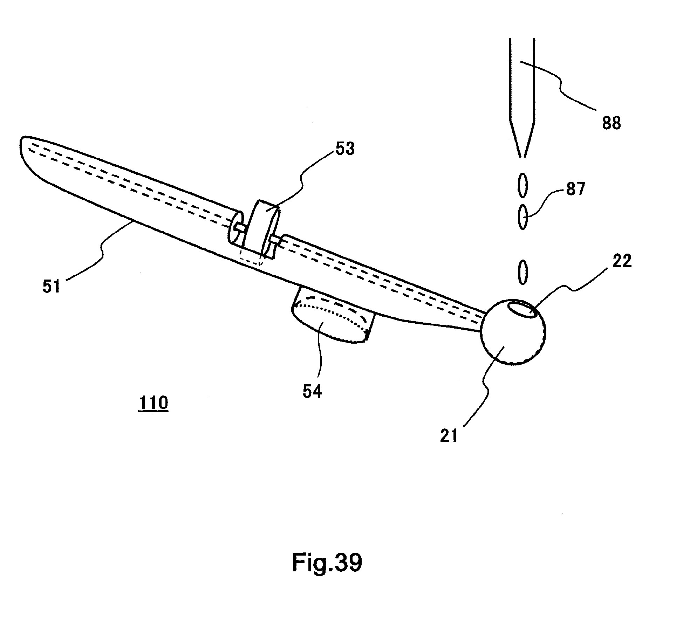

First, a mucous membrane is collected from a nostril by using the biological tissue collecting tool 110 in a manner similar to the above-described manner. Thereafter, as shown in Fig. 39, with the first collection opening 22 of the exterior case 21 facing upward, the operating portion 53 is rotated such that the position of the first collection opening 22 of the exterior case 21 and the position of the third collection opening 24 of the collecting portion 23 coincide with each other.

Next, with the use of a dropper 88, the reaction body 87 in liquid form is flowed into the collecting portion 23. As a result, an aggregate of amyloid beta is decomposed into simple substances. Then, with the use of another dropper (not shown), the neutralizer 86 in liquid form is flowed into the collecting portion 23 to neutralize the reaction body 87. Thereafter, the operating portion 53 is rotated again, so that the first collection opening 22 of the exterior case 21 is closed by the wall of the collecting portion 23.

Subsequently, similar to Embodiment 10, the push-in operating portion 84 is used to cause the distal end of the reagent body 81 to protrude into the collecting portion 23, and then the state of the reaction of the reagent is observed through the reaction confirmation window 85.

### Industrial Applicability

According to the present invention, a substantially fixed amount of biological tissue can be collected each time biological tissue, for example, a nostril mucous membrane, is collected from a biological aperture such as a nostril, and therefore, a variation in the amount of collected biological tissue can be reduced. This consequently makes it possible to improve the accuracy of a determination made in a test.

### Reference Signs List

- 10, 20, 30, 50, 60, 70, 100, 110: biological tissue collecting tool
- 1: external nostril
- 2: first collection opening
- 3: exterior body
- 4: operated body
- 5: third collection opening
- 6: storage portion
- 7: operating portion
- 8: interior body
- 9: second collection opening
- 11: common nasal meatus
- 12: upper space
- 13: third collection opening
- 14: packing
- 15: collecting portion
- 16: contacting portion
- 21: exterior case
- 22: first collection opening
- 23: collecting portion
- 24: third collection opening
- 25: retention holder
- 26: operating stick
- 27: packing
- 29: mucous membrane
- 31: beaker
- 32: liquid
- 33: dropper
- 34a, 34b: rotation restriction portion
- 35a: rotation restriction portion
- 36: insertion assisting tool
- 36a: cylindrical guide portion
- 37: storage case
- 38: external cylinder
- 39: internal cylinder
- 51: retention holder
- 52: notch
- 53: operating portion
- 54: finger rest
- 55: index finger
- 56: grip
- 61: wipe cover
- 62: tearing beginning point
- 63: diameter expanding portion
- 71: air hole
- 72: first rotating piece
- 73: second rotating piece
- 72a: first edge
- 72b: second edge
- 73a: first edge
- 73b: second edge
- 74: space
- 74: storage space
- 75: scratching portion
- 76: protrusion
- 77: groove
- 81: reagent body
- 84: push-in operating portion
- 85: reaction confirmation window
- 86: neutralizer
- 87: reaction body
- 88: dropper

## Claims

1. A biological tissue collecting tool comprising:
an exterior body having first space formed therein and having a first collection opening formed in a wall thereof, the exterior body having such a shape as to be insertable in a biological aperture;
a collecting portion disposed in the first space such that the collecting portion is movable in the first space, the collecting portion being configured to collect a part of biological tissue that protrudes into the first space through the first collection opening; and
an operating portion connected to the collecting portion, the operating portion allowing the collecting portion to be operated from outside of the exterior body.

2. The biological tissue collecting tool according to claim 1, wherein the exterior body is a cylindrical body having a closed end and an open end.

3. The biological tissue collecting tool according to claim 2, wherein the collecting portion is movable in the first space in an axial direction of the cylindrical exterior body.

4. The biological tissue collecting tool according to claim 2, wherein the collecting portion has a third collection opening formed in a wall thereof, and includes a storage portion configured to store the collected biological tissue, the storage portion being formed continuously with the third collection opening.

5. The biological tissue collecting tool according to claim 2, wherein the exterior body is formed such that the wall of the exterior body at an opposite side to the first collection opening is thicker than the wall of the exterior body that is positioned above and below the first collection opening with respect to the axial direction of the cylindrical exterior body.

6. The biological tissue collecting tool according to claim 2, wherein the exterior body is formed of a soft material whose hardness measured by a former JIS K 6301 spring-type hardness test is less than or equal to 30°.

7. The biological tissue collecting tool according to claim 6, wherein the hardness of the soft material is in a range from 5° to 10°.

8. The biological tissue collecting tool according to claim 6, wherein the soft material is formed of a silicone.

9. The biological tissue collecting tool according to claim 6, further comprising a cylindrical interior body disposed within the exterior body, the interior body having second space formed therein, the second space allowing the collecting portion to be movably disposed therein, the interior body having a second collection opening formed in a wall thereof and having a closed end and an open end.

10. The biological tissue collecting tool according to claim 9, further comprising packing which is disposed between the collecting portion and the operating portion so as to be in contact with an inner wall surface of the interior body.

11. The biological tissue collecting tool according to claim 10, wherein
the collecting portion includes a contacting portion configured to come into contact with the inner wall surface of the interior body at the closed end.

12. The biological tissue collecting tool according to claim 1, wherein each of the exterior body and the collecting portion is spherical and has a hollow center, and the collecting portion is rotatable in the first space.

13. A biological tissue collection method comprising:
inserting the biological tissue collecting tool according to any one of claims 1 to 12 into a common nasal meatus through an external nostril; and then
operating the operating portion to move the collecting portion and collect, by the collecting portion, a part of tissue in the common nasal meatus.
